(19) Europäisches Patentamt
European Patent Office
Office européen des brevets

(11) Veröffentlichungsnummer: **0 369 944 A1**

(12) **EUROPÄISCHE PATENTANMELDUNG**

(21) Anmeldenummer: **89810859.2**

(22) Anmeldetag: **09.11.89**

(51) Int. Cl.[5]: **A61K 31/15, C07C 251/58, C07C 239/20, C07C 309/66, C07D 213/69, C07D 215/12, C07F 9/58, C07H 5/04**

(30) Priorität: **18.11.88 CH 4276/88**

(43) Veröffentlichungstag der Anmeldung: **23.05.90 Patentblatt 90/21**

(84) Benannte Vertragsstaaten: **AT BE CH DE ES FR GB GR IT LI LU NL SE**

(71) Anmelder: **CIBA-GEIGY AG**
**Klybeckstrasse 141**
**CH-4002 Basel(CH)**

(72) Erfinder: **Stanek, Jaroslav, Dr.**
**Hangstrasse 9**
**CH-4144 Arlesheim(CH)**
Erfinder: **Caravatti, Giorgio, Dr.**
**Kurzelängeweg 30**
**CH-4123 Allschwil(CH)**
Erfinder: **Frei, Jörg, Dr.**
**Büchring 36**
**CH-4434 Hölstein(CH)**
Erfinder: **Capraro, Hans-Georg, Dr.**
**Habsburgerstrasse 60**
**CH-4310 Rheinfelden(CH)**

(54) **Substituierte Oxadiaminobutane.**

(57) Verbindungen der Formel I

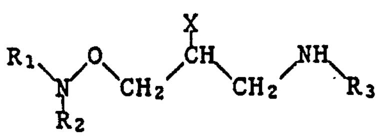

(I),

worin $R_1$, $R_2$, $R_3$ und X die in der Beschreibung angegebenen Bedeutungen haben, weisen wertvolle pharmazeutische Eigenschaften auf und sind insbesondere gegen Tumoren wirksam. Sie werden in an sich bekannter Weise hergestellt.

EP 0 369 944 A1

Xerox Copy Centre

## Substituierte Oxadiaminobutane

Die Erfindung betrifft die Verwendung von Verbindungen der Formel I

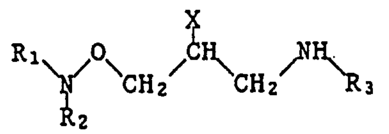

(I)

worin X Halogen, Hydroxy, Niederalkoxy, Acyloxy, Niederalkylsulfonyloxy oder Arylsulfonyloxy bedeutet; $R_1$ und $R_2$ unabhängig voneinander Wasserstoff, Niederalkyl, Cycloalkyl, Arylniederalkyl oder Hetarylniederalkyl bedeuten, oder $R_1$ und $R_2$ zusammen für einen Rest

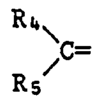

stehen, worin $R_4$ und $R_5$ unabhängig voneinander Wasserstoff, Niederalkyl, Hydroxyniederalkyl, Halogenniederalkyl, Niederalkoxyniederalkyl, Niederalkenyl, Niederalkinyl, Cycloalkyl, Arylniederalkyl, Hetarylniederalkyl, Aryl, Hetaryl, Carboxy oder Niederalkoxycarbonyl bedeuten, oder worin $R_4$ Polyhydroxyniederalkyl bedeutet und $R_5$ für Wasserstoff oder Hydroxymethyl steht, oder worin $R_4$ und $R_5$ zusammen $C_2$-$C_7$-Alkylen oder gegebenenfalls substituiertes Benzo-$C_4$-$C_6$-alkylen bedeuten; und $R_3$ für Wasserstoff, gegebenenfalls durch Hydroxy oder Halogen und/oder Aminoxy substituiertes Niederalkyl, Niederalkenyl, Niederalkinyl oder gegebenenfalls verethertes Hydroxy steht; und pharmazeutisch annehmbaren Salzen davon, (zur Herstellung von pharmazeutischen Präparaten) zur therapeutischen Behandlung von Krankheiten des menschlichen oder tierischen Körpers, die auf eine Hemmung der Ornithindecarboxylase ansprechen, wie zum Beispiel Tumoren oder Protozoainfektionen, und ferner neue Verbindungen der Formel I.

Die Erfindung betrifft ferner die Verbindungen der Formel I

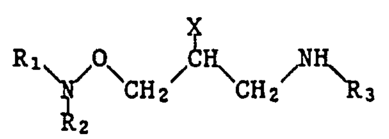

(I),

worin X Halogen, Hydroxy, Niederalkoxy, Acyloxy, Niederalkylsulfonyloxy oder Arylsulfonyloxy bedeutet; $R_1$ und $R_2$ unabhängig voneinander Wasserstoff, Niederalkyl, Cycloalkyl, Arylniederalkyl oder Hetarylniederalkyl bedeuten, oder $R_1$ und $R_2$ zusammen für einen Rest

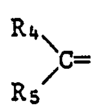

stehen, worin $R_4$ und $R_5$ unabhängig voneinander Wasserstoff, Niederalkyl, Hydroxyniederalkyl, Halogenniederalkyl, Niederalkoxyniederalkyl, Niederalkenyl, Niederalkinyl, Cycloalkyl, Arylniederalkyl, Hetarylniederalkyl, Aryl, Hetaryl, Carboxy oder Niederalkoxycarbonyl bedeuten, oder worin $R_4$ Polyhydroxyniederalkyl bedeutet und $R_5$ für Wasserstoff oder Hydroxymethyl steht, oder worin $R_4$ und $R_5$ zusammen $C_2$-$C_7$-Alkylen oder gegebenenfalls substituiertes Benzo-$C_4$-$C_6$-alkylen bedeuten; und $R_3$ für Wasserstoff, gegebenenfalls durch Hydroxy oder Halogen und/oder Aminoxy substituiertes Niederalkyl, Niederalkenyl, Niederalkinyl oder gegebenenfalls verethertes Hydroxy steht;
mit der Massgabe, dass, wenn X Hydroxy bedeutet und $R_1$ und $R_2$ zusammen für einen Rest

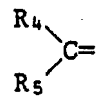

stehen, $R_3$ Wasserstoff bedeutet;

mit der Massgabe, dass, wenn X für Hydroxy steht und $R_3$ Wasserstoff bedeutet, die Reste $R_1$ und $R_2$ zusammen nicht 1,1-Dimethylmethyliden, 1-n-Butyliden oder Nitro-(furanyl oder imidazolyl)-methyliden bedeuten können;

mit der Massgabe, dass, wenn X für Hydroxy steht und $R_3$ Wasserstoff, Hydroxy oder Niederalkyl bedeutet, nicht beide Reste $R_1$ und $R_2$ Wasserstoff bedeuten können;

und mit der Massgabe, dass, wenn X Hydroxy bedeutet und $R_1$ und $R_2$ unabhängig voneinander Wasserstoff, Niederalkyl, Cycloalkyl, Arylniederalkyl oder Hetarylniederalkyl, nicht jedoch beide Wasserstoff, bedeuten, $R_3$ für Wasserstoff, Methyl, Ethyl oder n-Propyl steht;

und ihre Salze, Verfahren zur Herstellung dieser Verbindungen, pharmazeutische Präparate, die diese Verbindungen enthalten, die Verwendung dieser Verbindungen zur therapeutischen Behandlung des menschlichen oder tierischen Körpers oder zur Herstellung pharmazeutischer Präparate.

Die vor- und nachstehend verwendeten Allgemeinbegriffe haben im Rahmen der vorliegenden Anmeldung die folgenden Bedeutungen:

Das Präfix "Nieder" bezeichnet einen Rest bis und mit 7 und insbesondere bis und mit 4 Kohlenstoffatomen.

Niederalkyl ist z.B. n-Propyl, Isopropyl, n-Butyl, Isobutyl, sek-Butyl, tert-Butyl, n-Pentyl, Neopentyl, n-Hexyl oder n-Heptyl, vorzugsweise Ethyl und vor allem Methyl.

Niederalkenyl ist z.B. Vinyl, Allyl, 1-Propenyl, Isopropenyl, 2- oder 3-Methallyl oder 3-Butenyl.

Niederalkinyl ist z.B. Ethinyl oder 2-Butinyl, und insbesondere Propargyl.

Cycloalkyl enthält z.B. 3 bis 8 und in erster Linie 5 oder 6 Ringkohlenstoffatome und ist z.B. Cyclopropyl, Cyclopentyl, Cyclohexyl oder Cycloheptyl.

Verethertes Hydroxy ist z.B. Niederalkoxy oder Phenylniederalkoxy.

Niederalkoxy ist z.B. n-Propoxy, Isopropoxy, n-Butoxy oder tert.-Butoxy, vorzugsweise Ethoxy, und vor allem Methoxy, während Phenylniederalkoxy z.B. Benzyloxy ist.

Acyloxy ist vorzugsweise Niederalkanoyloxy, ferner aber auch z.B. Arylcarbonyloxy (= Aroyloxy) oder Hetarylcarbonyloxy (= Hetaroyloxy).

Arylcarbonyloxy ist z.B. Benzoyloxy.

Hetarylcarbonyloxy ist z.B. Pyridoyloxy oder Thienoyloxy.

Niederalkylsulfonyloxy ist z.B. Methylsulfonyloxy.

Arylsulfonyloxy ist z.B. gegebenenfalls durch Niederalkyl oder Halogen substituiertes Phenylsulfonyloxy, wie Phenyl- oder 4-Methylphenylsulfonyloxy.

Niederalkoxycarbonyl ist z.B. Propoxycarbonyl oder Butoxycarbonyl, vorzugsweise Methoxycarbonyl und Ethoxycarbonyl.

Halogen im allgemeinen bedeutet z.B. Fluor oder Iod, insbesondere Brom und vor allem Chlor.

Ein Halogenrest X in einer Verbindung der Formel I ist insbesondere Chlor und in erster Linie Fluor, kann aber z.B. auch für Brom oder Iod stehen.

Halogenniederalkyl ist z.B. Difluormethyl oder Trifluormethyl, ferner 1-Chlorethyl.

Hydroxyniederalkyl ist z.B. Hydroxymethyl oder 2-Hydroxyethyl.

Niederalkoxyniederalkyl ist z.B. Methoxymethyl.

Polyhydroxyniederalkyl ist z.B. 1,2-Dihydroxyethyl oder 1,2,3-Trihydroxypropyl, vorzugsweise 1,2,3,4-Tetrahydroxybutyl und insbesondere 1,2,3,4,5-Pentahydroxypentyl.

Durch Hydroxy oder Halogen und/oder Aminoxy substituiertes Niederalkyl ist z.B. Hydroxyniederalkyl oder Halogenniederalkyl, ferner Hydroxyaminoxy-niederalkyl, z.B. 3-Aminoxy-2-hydroxy-propyl, oder Hydroxyhalogen-niederalkyl, z.B. 3-Aminoxy-2-fluor-propyl.

Verbindungen der Formel I, worin $R_1$ und $R_2$ zusammen für einen Rest $R_5R_4C=$ stehen, $R_4$ Polyhydroxyniederalkyl bedeutet und $R_5$ für Wasserstoff oder Hydroxymethyl steht, leiten sich bevorzugt von Zuckern, insbesondere Aldo- bzw. Keto-(triosen, tetrosen, pentosen oder hexosen) ab. Vor allen Dingen bevorzugt ist dabei der Rest $R_5R_4C=$, worin $R_4$ 1,2,3,4,5-Pentahydroxypentyl (von der D-Glucose abgeleitet) und $R_5$ Wasserstoff bedeutet.

Niederalkanoyl ist z.B. Formyl, Propionyl oder Butyryl, und vor allem Acetyl.

Niederalkanoyloxy ist z.B. Formyloxy, Propionyloxy oder Butyryloxy, und vor allem Acetyloxy.

Aryl ist z.B. Phenyl oder Naphthyl, wie 1- oder 2-Naphthyl. Die Phenyl-oder Naphthylreste können

unsubstituiert oder substituiert sein. Aryl ist bevorzugt Phenyl, das unsubstituiert oder durch Niederalkyl, Halogenniederalkyl, Hydroxyniederalkyl, Halogen, Hydroxy, Niederalkoxy, Niederalkanoyloxy und/oder Nitro substituiert ist, und in erster Linie Phenyl.

Arylniederalkyl ist vorzugsweise Phenylniederalkyl und insbesondere Benzyl.

Unter Hetaryl - als solchem oder in zusammengesetzten Begriffen wie z.B. Hetarylniederalkyl - ist ein insbesondere monozyklischer, aber auch bi-oder polyzyklischer, heterozyklischer Rest aromatischen Charakters zu verstehen. Bi- und polyzyklisches Hetaryl kann aus mehreren heterozyklischen Ringen zusammengesetzt sein oder bevorzugt aus einem Heterozyklus und einem oder mehreren, z.B. einem oder zwei und insbesondere einem, annellierten carbozyklischen Ring, insbesondere-Benzoring, bestehen.

Jeder einzelne Ring enthält z.B. 3, 5, 6 oder 7 und insbesondere 5 oder 6 Ringglieder. Hetaryl steht insbesondere für einen aza-, thia-, oxa-, thiaza-, thiadiaza-, oxaza-, diaza-, triaza- und tetrazacyclischen Rest.

Hetaryl steht in erster Linie für monocyclische monoaza-, monothia- oder monooxacyclische Reste, wie etwa Pyrryl, z.B. 2-Pyrryl oder 3-Pyrryl, Pyridyl, z.B. 2-, 3- oder 4-Pyridyl, Thienyl, z.B. 2- oder 3-Thienyl, oder Furyl, z.B. 2-Furyl; bicyclische monoaza-, monooxa- oder monothiacyclische Reste, wie Indolyl, z.B. 2- oder 3-Indolyl, Chinolinyl, z.B. 2- oder 4-Chinolinyl, Isochinolinyl, z.B. 1-Isochinolinyl, Benzofuranyl, z.B. 2- oder 3-Benzofuranyl, oder Benzothienyl, z.B. 2- oder 3-Benzothienyl; monocyclische diaza-, triaza-, tetraza-, oxaza-, thiaza-oder thiadiazacyclische Reste, wie Imidazolyl, z.B. 2-Imidazolyl, Pyrimidinyl, z.B. 2- oder 4-Pyrimidinyl, Triazolyl, z.B. 1,2,4-Triazol-3-yl, Tetrazolyl, z.B. 1- oder 5-Tetrazolyl, Oxazolyl, z.B. 2-Oxazolyl, Isoxazolyl, z.B. 3- oder 4-Isoxazolyl, Thiazolyl, z.B. 2-Thiazolyl, Isothiazolyl, z.B. 3- oder 4-Isothiazolyl oder 1,2,4- oder 1,3,4-Thiadiazolyl, z.B. 1,2,4-Thiadiazol-3-yl oder 1,3,4-Thiadiazol-2-yl; oder bicyclische diaza-, oxaza- oder thiazacyclische Reste, wie Benzimidazolyl, z.B. 2-Benzimidazolyl, Benzoxazolyl, z.B. 2-Benzoxazolyl, oder Benzthiazolyl, z.B. 2-Benzthiazolyl.

Hetarylreste sind unsubstituiert oder tragen Substituenten. Als Substituenten an Ringkohlenstoffatomen kommen z.B. die oben für Arylreste angegebenen Substituenten in Frage und zusätzlich Phosphonooxymethyl[$-CH_2-O-P(=O)(OH)_2$], ferner Oxo (=O). Ringstickstoffatome können z.B. durch Niederalkyl, Arylniederalkyl, Niederalkanoyl, Benzoyl, Carboxy, Niederalkoxycarbonyl, Hydroxy, Niederalkoxy, Niederalkanoyloxy oder Oxido

$$(-\bar{\underline{O}}|)$$

substituiert sein.

Hetaryl ist vor allen Dingen Pyridyl, Chinolinyl, Thienyl, Pyrryl oder Imidazolyl.

Hetarylniederalkyl ist vor allen Dingen Pyridyl-, Thienyl-, Pyrryl- oder Imidazolylmethyl.

Unter einem Rest $R_5R_4C=$, worin $R_4$ und $R_5$ zusammen $C_2$-$C_7$-Alkylen bedeuten, ist Cycloalkyliden mit 3 bis 8 Ringkohlenstoffatomen. z.B. Cyclopropyliden, Cyclopentyliden oder Cyclohexyliden, zu verstehen.

Unter einem Rest $R_5R_4C=$, worin $R_4$ und $R_5$ zusammen gegebenenfalls substituiertes Benzo-$C_4$-$C_6$-alkylen bedeuten, ist z.B. Cyclopentyliden oder Cyclohexyliden zu verstehen, welche jeweils einen annellierten Benzoring tragen, welcher unsubstituiert oder durch Niederalkyl, Niederalkoxy, Carboxy, Niederalkoxycarbonyl, Carbamoyl und/oder Cyano substituiert ist. Beispiele für solche Reste sind Indan-1-yliden, 4-Cyano-indan-1-yliden und 1,2,3,4-Tetrahydro-1-naphthyliden.

Ein Rest $R_5R_4C=$, worin $R_4$ 3-Hydroxy-5-(hydroxymethyl oder phosphonooxymethyl)-2-methyl-4-pyridyl bedeutet und $R_5$ für Wasserstoff steht bzw. ein aus $R_1$ und $R_2$ zusammen gebildeten Rest 3-Hydroxy-5-(hydroxymethyl oder phosphonooxymethyl)-2-methyl-4-pyridylmethyliden ist vom Pyridoxal bzw. Pyridoxal-5-phosphat abgeleitet. Ein Phosphono-Rest entspricht der Gruppe $-P(=O)(OH)_2$.

$C_1$-$C_4$-Alkyliden ist bevorzugt Ethyliden oder 1,1-Dimethylmethyliden [$=C(CH_3)_2$], ferner auch z.B. 1-n-Butyliden [$=CH-CH_2-CH_2-CH_3$] oder 2-n-Butyliden [$CH_3-C(=)-CH_2-CH_3$].

Salze von erfindungsgemässen Verbindungen sind in erster Linie pharmazeutisch annehmbare, nichttoxische Salze. Beispielsweise können Verbindungen der Formel I mit basischen Gruppen Säureadditionssalze, z.B. mit anorganischen Säuren, wie Salzsäure, Schwefelsäure oder Phosphorsäure, oder mit geeigneten organischen Carbon- oder Sulfonsäuren, z.B. Essigsäure, Fumarsäure, Oxalsäure oder Methansulfonsäure, oder z.B. mit Aminosäuren, wie Arginin oder Lysin, bilden. Bei Anwesenheit von mehreren basischen Gruppen können Mono- oder Polysalze gebildet werden. Verbindungen der Formel I mit einer sauren Gruppe, z.B. Carboxy, und einer basischen Gruppe, z.B. Amino, können z.B. in Form von inneren Salzen, d.h. in zwitterionischer Form vorliegen, oder es kann ein Teil des Moleküls als inneres Salz, und ein anderer Teil als normales Salz vorliegen.

Zur Isolierung oder Reinigung können auch pharmazeutisch ungeeignete Salze, z.B. Pikrate oder

Perchlorate, Verwendung finden. Zur therapeutischen Anwendung gelangen nur die pharmazeutisch annehmbaren, nichttoxischen Salze, die deshalb bevorzugt sind.

Je nach den strukturellen Gegebenheiten können die Verbindungen der vorliegenden Erfindung in Form von Isomerengemischen oder von reinen Isomeren vorliegen.

Die erfindungsgemässen Verbindungen weisen wertvolle, insbesondere pharmakologisch verwendbare, Eigenschaften auf. Insbesondere haben sie eine starke, spezifische Hemmwirkung auf das Enzym Ornithindecarboxylase (ODC). ODC spielt als ein Schlüsselenzym eine wichtige Rolle bei der Polyaminsynthese, die in praktisch allen Zellen von Säugetieren, einschliesslich Menschen, abläuft. Durch ODC wird die Polyaminkonzentration in der Zelle reguliert. Eine Hemmung des Enzyms ODC hat eine Verringerung der Polyaminkonzentration zur Folge. Da eine Verringerung der Polyaminkonzentration eine Hemmung des Zellwachstums bewirkt, ist es möglich, durch Verabreichung von ODC-hemmenden Substanzen das Wachstum sowohl von eukaryotischen als auch prokaryotischen, inbesondere von rasch oder unkontrollierbar wachsenden, Zellen zu hemmen und sogar Zellen abzutöten oder das Einsetzen der Zelldifferenzierung zu hemmen.

Die Hemmung des Enzyms ODC kann z.B. mit der Methode von J.E. Seely und A.E. Pegg, Ornithin decarboxylase (mouse kidney), Seiten 158-161, in H. Tabor und C. White Tabor (Hrsg.): Methods in enzymology, Vol. 94: Polyamines, Academic Press, New York 1983, nachgewiesen werden. Die Verbindungen der Erfindung weisen $IC_{50}$-Werte von minimal etwa 0.01 $\mu$M auf.

Die Verbindungen der Formel I besitzen antiproliferative Eigenschaften, die sich z.B. in dem folgenden Versuch direkt demonstrieren lassen: Dabei wird die Hemmwirkung der Verbindungen der Formel I auf das Wachstum von menschlichen T24 Blasenkarzinomzellen bestimmt. Diese Zellen werden in "Eagle's minimal essential medium", dem 5 % (V/V) fötales Kälberserum zugesetzt sind, in einem befeuchteten Inkubator bei 37° C und 5 Volumenprozent $CO_2$ in der Luft inkubiert. Die Karzinomzellen (1000-1500) werden in 96-Loch-Mikrotiterplatten überimpft und über Nacht unter den obengenannten Bedingungen inkubiert. Die Testsubstanz wird in seriellen Verdünnungen am Tag 1 hinzugefügt. Die Platten werden unter den obengenannten Bedingungen 5 Tage lang inkubiert. Während dieser Zeitspanne durchlaufen die Kontrollkulturen mindestens 4 Zellteilungen. Nach der Inkubation werden die Zellen mit 3,3%iger (Gewicht/Volumen) wässriger Glutaraldehydlösung fixiert, mit Wasser gewaschen und mit 0,05%iger (G/V) wässriger Methylenblaulösung gefärbt. Nach dem Waschen wird der Farbstoff mit 3%iger (G/V) wässriger Salzsäure eluiert. Danach wird die optische Dichte (OD) pro Loch, welche der Zellanzahl direkt proportinal ist, mit einem Photometer (Titertek multiskan) bei 665 nm gemessen. Die $IC_{50}$-Werte werden mit einem Computersystem unter Verwendung der Formel

$$\frac{OD_{665}\ (\text{Test}) - OD_{665}\ (\text{Anfang})}{OD_{665}\ (\text{Kontrolle}) - OD_{665}\ (\text{Anfang})} \times 100$$

errechnet. Die $IC_{50}$-Werte sind als diejenige Wirkstoffkonzentration definiert, bei der die Anzahl der Zellen pro Loch am Ende der Inkubationszeit nur 50 % der Zellanzahl in den Kontrollkulturen beträgt. Die so ermittelten $IC_{50}$-Werte liegen für die Verbindungen der Formel I minimal bei etwa 1 $\mu$M.

Die Verbindungen der Formel I sind daher z.B. nützlich zur Behandlung benigner und maligner Tumoren. Sie können Tumorregressionen bewirken und ferner die Verbreitung von Tumorzellen sowie das Wachstum von Mikrometastasen verhindern. Des weiteren können sie z.B. zur Behandlung von Protozoainfektionen, wie etwa Trypanosomiasis, Malaria oder durch Pneumocystis carinii verursachte Lungenentzündung, dienen.

Bevorzugt betrifft die Erfindung die oben angegebene Verwendung von Verbindungen der Formel I, worin X Halogen, Hydroxy, Niederalkoxy, Niederalkylsulfonyloxy oder Phenylsulfonyloxy bedeutet; $R_1$ und $R_2$ unabhängig voneinander Wasserstoff, Niederalkyl, $C_3$-$C_8$-Cycloalkyl oder Phenylniederalkyl bedeuten, oder $R_1$ und $R_2$ zusammen für einen Rest

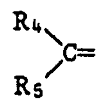

stehen, worin $R_4$ und $R_5$ unabhängig voneinander Wasserstoff, Niederalkyl, Halogenniederalkyl, $C_3$-$C_8$-Cycloalkyl, Phenylniederalkyl oder Phenyl bedeuten, oder worin $R_4$ 3-Hydroxy5-(hydroxymethyl oder phosphonooxymethyl)-2-methyl-4-pyridyl bedeutet und $R_5$ für Wasserstoff steht, oder worin $R_4$ 1,2,3,4-Tetrahydroxybutyl oder 1,2,3,4,5-Pentahydroxypentyl bedeutet und $R_5$ für Wasserstoff oder Hydroxymethyl steht, oder worin $R_4$ Chinolinyl, Carboxy oder Niederalkoxycarbonyl bedeutet und $R_5$ für Wasserstoff oder Niederalkyl steht, oder worin $R_4$ und $R_5$ zusammen $C_2$-$C_7$-Alkylen oder gegebenenfalls durch Cyano substituiertes Benzo-$C_4$-$C_5$-alkylen bedeuten; und $R_3$ für Wasserstoff, Niederalkyl, Niederalkenyl, Niederalkinyl, Niederalkoxy, Benzyloxy oder Hydroxy steht; wobei Phenylgruppen unsubstituiert oder durch Niederalkyl, Halogenniederalkyl, Halogen, Hydroxy, Niederalkoxy, Niederalkanoyloxy und/oder Nitro substituiert sind; und ihren pharmazeutisch annehmbaren Salzen.

Insbesondere betrifft die Erfindung die oben angegebene Verwendung von Verbindungen der Formel I, worin X Fluor, Chlor, Hydroxy, Niederalkylsulfonyloxy oder Phenylsulfonyloxy bedeutet; $R_1$ und $R_2$ unabhängig voneinander Wasserstoff, Niederalkyl, $C_3$-$C_7$-Cycloalkyl oder Phenylniederalkyl bedeuten, oder $R_1$ und $R_2$ zusammen für einen Rest

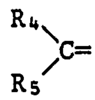

stehen, worin $R_4$ und $R_5$ unabhängig voneinander Wasserstoff, Niederalkyl, Halogenniederalkyl, $C_3$-$C_7$-Cycloalkyl, Phenylniederalkyl oder Phenyl bedeuten, oder worin $R_4$ Chinolinyl, 3-Hydroxy-5-(hydroxymethyl oder phosphonooxymethyl)-2-methyl-4-pyridyl bedeutet und $R_5$ für Wasserstoff steht, oder worin $R_4$ Carboxy oder Niederalkoxycarbonyl bedeutet und $R_5$ für Niederalkyl steht, oder worin $R_4$ und $R_5$ zusammen $-(CH_2)_4-$ oder $-(CH_2)_5-$ bedeuten; und $R_3$ für Wasserstoff, Niederalkyl, Niederalkenyl, Niederalkinyl oder Hydroxy steht; wobei Phenylgruppen unsubstituiert oder durch Niederalkyl, Trifluormethyl, Halogen, Hydroxy, Niederalkoxy und/oder Nitro substituiert sind; und ihren pharmazeutisch annehmbaren Salzen.

In erster Linie betrifft die Erfindung die oben angegebene Verwendung von Verbindungen der Formel I, worin X Fluor, Chlor, Hydroxy oder Methylsulfonyloxy bedeutet, $R_1$ für Wasserstoff steht, $R_2$ Wasserstoff, $C_1$-$C_4$-Alkyl, Benzyl oder 2-Hydroxybenzyl bedeutet, oder $R_1$ und $R_2$ zusammen $C_5$-$C_6$-Cycloalkyliden, $C_1$-$C_4$-Alkyliden, Benzyliden, 2-Hydroxybenzyliden oder 3-Hydroxy-5-(hydroxymethyl oder phosphonooxymethyl)-2-methyl-4-pyridylmethyliden bedeuten, und $R_3$ für Wasserstoff, $C_1$-$C_4$-Alkyl, $C_2$-$C_4$-Alkinyl oder Hydroxy steht; und ihren pharmazeutisch annehmbaren Salzen.

Insbesondere betrifft die Erfindung die oben angegebene Verwendung von 3-Aminoxy-2-hydroxypropylamin und 3-Aminoxy-2-fluoro-propylamin und pharmazeutisch annehmbaren Salzen davon.

Bevorzugt betrifft die Erfindung Verbindungen der Formel I, worin X Halogen, Hydroxy, Niederalkoxy, Niederalkylsulfonyloxy oder Phenylsulfonyloxy bedeutet; $R_1$ und $R_2$ unabhängig voneinander Wasserstoff, Niederalkyl, $C_3$-$C_8$-Cycloalkyl oder Phenylniederalkyl bedeuten, oder $R_1$ und $R_2$ zusammen für einen Rest

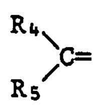

stehen, worin $R_4$ und $R_5$ unabhängig voneinander Wasserstoff, Niederalkyl, Halogenniederalkyl, $C_3$-$C_8$-Cycloalkyl, Phenylniederalkyl oder Phenyl bedeuten, oder worin $R_4$ 3-Hydroxy-5-(hydroxymethyl oder phosphonooxymethyl)-2-methyl-4-pyridyl bedeutet und $R_5$ für Wasserstoff steht, oder worin $R_4$ 1,2,3,4-Tetrahydroxybutyl oder 1,2,3,4,5-Pentahydroxypentyl bedeutet und $R_5$ für Wasserstoff oder Hydroxymethyl steht, oder worin $R_4$ Chinolinyl, Carboxy oder Niederalkoxycarbonyl bedeutet und $R_5$ für Wasserstoff oder Niederalkyl steht, oder worin $R_4$ und $R_5$ zusammen $C_2$-$C_7$-Alkylen oder gegebenenfalls durch Cyano substituiertes Benzo-$C_4$-$C_6$-alkylen bedeuten; und $R_3$ für Wasserstoff, Niederalkyl, Niederalkenyl, Niederalkinyl, Niederalkoxy, Benzyloxy oder Hydroxy steht;
mit der Massgabe, dass, wenn X Hydroxy bedeutet und $R_1$ und $R_2$ zusammen für einen Rest

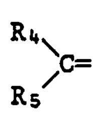

stehen, $R_3$ Wasserstoff bedeutet;
mit der Massgabe, dass, wenn X für Hydroxy steht und $R_3$ Wasserstoff bedeutet, die Reste $R_1$ und $R_2$ zusammen nicht 1,1-Dimethylmethyliden oder 1-n-Butyliden bedeuten können;

mit der Massgabe, dass, wenn X für Hydroxy steht und $R_3$ Wasserstoff, Hydroxy oder Niederalkyl bedeutet, nicht beide Reste $R_1$ und $R_2$ Wasserstoff bedeuten können;
und mit der Massgabe, dass, wenn X Hydroxy bedeutet und $R_1$ und $R_2$ unabhängig voneinander Wasserstoff, Niederalkyl, Cycloalkyl oder Phenylniederalkyl, nicht jedoch beide Wasserstoff, bedeuten, $R_3$ für Wasserstoff, Methyl, Ethyl oder n-Propyl steht;
wobei Phenylgruppen unsubstituiert oder durch Niederalkyl, Halogenniederalkyl, Halogen, Hydroxy, Niederalkoxy, Niederalkanoyloxy und/oder Nitro substituiert sind; und ihre Salze.

Bevorzugt betrifft die Erfindung Verbindungen der Formel I, worin X Fluor, Chlor, Hydroxy, Niederalkylsulfonyloxy oder Phenylsulfonyloxy bedeutet; $R_1$ und $R_2$ unabhängig voneinander Wasserstoff, Niederalkyl, $C_3$-$C_7$-Cycloalkyl oder Phenylniederalkyl bedeuten, mit der Massgabe, dass nicht beide Reste $R_1$ und $R_2$ Wasserstoff bedeuten können, wenn X für Hydroxy steht,
oder worin $R_1$ und $R_2$ zusammen für einen Rest

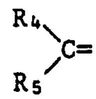

stehen, worin $R_4$ und $R_5$ unabhängig voneinander Wasserstoff, Niederalkyl, Halogenniederalkyl, $C_3$-$C_7$-Cycloalkyl, Phenylniederalkyl oder Phenyl bedeuten, oder worin $R_4$ Chinolinyl, 3-Hydroxy-5-(hydroxymethyl oder phosphonooxymethyl)-2-methyl-4-pyridyl bedeutet und $R_5$ für Wasserstoff steht, oder worin $R_4$ Carboxy oder Niederalkoxycarbonyl bedeutet und $R_5$ für Niederalkyl steht, oder worin $R_4$ und $R_5$ zusammen $-(CH_2)_4-$ oder $-(CH_2)_5-$ bedeuten; und $R_3$ für Wasserstoff steht;
mit der Massgabe, dass, wenn X für Hydroxy steht, die Reste $R_1$ und $R_2$ zusammen nicht 1,1-Dimethylmethyliden oder 1-n-Butyliden bedeuten können;
wobei Phenylgruppen unsubstituiert oder durch Niederalkyl, Trifluormethyl, Halogen, Hydroxy, Niederalkoxy oder Niederalkanoyloxy substituiert sind; und ihre Salze.

In erster Linie betrifft die Erfindung Verbindungen der Formel I, worin X Fluor, Chlor oder Methylsulfonyloxy bedeutet; $R_1$ und $R_2$ unabhängig voneinander Wasserstoff, Niederalkyl, $C_3$-$C_7$-Cycloalkyl oder Phenylniederalkyl bedeuten, oder $R_1$ und $R_2$ zusammen für einen Rest

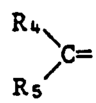

stehen, worin $R_4$ und $R_5$ unabhängig voneinander Wasserstoff, Niederalkyl, Halogenniederalkyl, $C_3$-$C_7$-Cycloalkyl, Phenylniederalkyl oder Phenyl bedeuten, oder worin $R_4$ Chinolinyl, 3-Hydroxy-5-(hydroxymethyl oder phosphonooxymethyl)-2-methyl-4-pyridyl bedeutet und $R_5$ für Wasserstoff steht, oder worin $R_4$ Carboxy oder Niederalkoxycarbonyl bedeutet und $R_5$ für Niederalkyl steht, oder worin $R_4$ und $R_5$ zusammen $-(CH_2)_4-$ oder $-(CH_2)_5-$ bedeuten; und $R_3$ für Wasserstoff, Niederalkyl, Niederalkenyl, Niederalkinyl oder Hydroxy steht; wobei Phenylgruppen unsubstituiert oder durch Niederalkyl, Trifluormethyl, Halogen, Hydroxy, Niederalkoxy und/oder Nitro substituiert sind; und ihre Salze.

Vor allen Dingen betrifft die Erfindung Verbindungen der Formel I, worin X Fluor, Chlor oder Methylsulfonyloxy bedeutet, $R_1$ für Wasserstoff steht, $R_2$ Wasserstoff, $C_1$-$C_4$-Alkyl, Benzyl oder 2-Hydroxybenzyl bedeutet, oder $R_1$ und $R_2$ zusammen $C_5$-$C_6$-Cycloalkyliden, $C_1$-$C_4$-Alkyliden, Benzyliden, 2-Hydroxybenzyliden oder 3-Hydroxy-5-(hydroxymethyl oder phosphonooxymethyl)-2-methyl-4-pyridylmethyliden bedeuten, und $R_3$ für Wasserstoff, $C_1$-$C_4$-Alkyl, $C_2$-$C_4$-Alkinyl oder Hydroxy steht; und ihre Salze.

Als Untergruppen aus einer Gruppe von Verbindungen der Formel I sind jeweils hervorzuheben:

(a) Verbindungen der Formel I, worin $R_1$ und $R_2$ Wasserstoff bedeuten und X für Halogen steht;

(b) Verbindungen der Formel I, worin $R_1$ und $R_2$ zusammen einen Rest

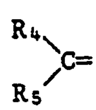

bedeuten;

(c) Verbindungen der Formel I, worin $R_3$ Wasserstoff bedeutet.

Die Erfindung betrifft vor allem die in den Beispielen beschriebenen spezifischen Verbindungen und pharmazeutisch annehmbare Salze davon.

Die neuen Verbindungen der Formel I können in an sich bekannter Weise hergestellt werden, indem man z.B.

(a) zur Herstellung von Verbindungen der Formel I, worin X für Halogen steht, eine Verbindung der Formel II

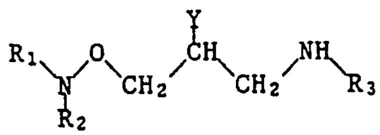 (II)

worin $R_1$, $R_2$ und $R_3$ die unter Formel I angegebene Bedeutung haben und Y eine in Halogen überführbare oder durch Halogen austauschbare Gruppe ist, mit einem Halogenierungsmittel umsetzt, oder

(b) zur Herstellung von Verbindungen der Formel I, worin X für Hydroxy steht, eine Verbindung der Formel III

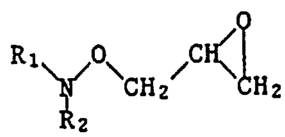 (III)

worin $R_1$ und $R_2$ die unter Formel I angegebene Bedeutung haben, mit Ammoniak bzw. einem Amin der Formel $NH_2R_3$, worin $R_3$ die unter Formel I angegebene Bedeutung hat, umsetzt, oder

(c) zur Herstellung von Verbindungen der Formel I, worin $R_1$ Wasserstoff bedeutet, in einer Verbindung der Formel IV

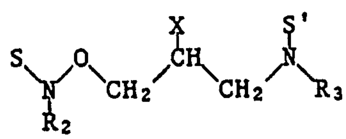 (IV)

worin $R_2$ und $R_3$ die unter Formel I angegebene Bedeutung haben und S und S′ unabhängig voneinander für eine Aminoschutzgruppe oder Wasserstoff stehen, wobei mindestens eine der Gruppen S und S′ eine Aminoschutzgruppe bedeutet, oder worin S und $R_2$ zusammen bzw. S′ und $R_3$ zusammen eine bivalente Aminoschutzgruppe bedeuten, die Aminoschutzgruppe(n) abspaltet, oder

(d) eine Verbindung der Formel V

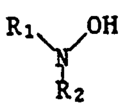 (V)

worin $R_1$ und $R_2$ die unter Formel I angegebene Bedeutung haben, mit einer Verbindung der Formel VI

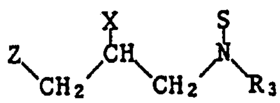 (VI)

worin $R_3$ und X die unter Formel I angegebene Bedeutung haben, Z Hydroxy oder eine nucleofuge Abgangsgruppe bedeutet und S eine Aminoschutzgruppe oder Wasserstoff bedeutet, umsetzt und gegebenenfalls die Aminoschutzgruppe abspaltet, oder

(e) zur Herstellung von Verbindungen der Formel I, worin $R_3$ Wasserstoff bedeutet, in einer Verbindung der Formel VII

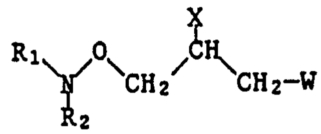

(VII)

worin $R_1$, $R_2$ und X die unter Formel I angegebene Bedeutung haben und W einen in Amino überführbaren Rest bedeutet, den Rest in in Amino überführt; oder

(f) zur Herstellung von Verbindungen der Formel I, worin $R_3$ Wasserstoff oder insbesondere Hydroxy oder Niederalkyl bedeutet, eine Verbindung der Formel VIII

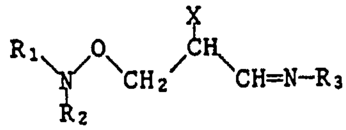

(VIII)

worin $R_1$, $R_2$ und X die unter Formel I angegebene Bedeutung haben und $R_3$ Wasserstoff, Hydroxy oder Niederalkyl bedeutet, reduziert;

und/oder wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung in ein Salz umwandelt, und/oder ein erhaltenes Gemisch von isomeren Verbindungen der Formel I in die einzelnen Isomeren auftrennt.

In der folgenden näheren Beschreibung der Verfahren (a)-(f) haben die Symbole X, $R_1$, $R_2$ und $R_3$ jeweils die unter Formel I angegebene Bedeutung, sofern nichts anderes angegeben ist.

Verfahren (a):

Eine in Halogen überführbare oder durch Halogen austauschbare Gruppe Y ist z.B. Hydroxy oder aliphatisch- oder aromatisch-substituiertes Sulfonyloxy, z.B. Methylsulfonyloxy oder 4-Methylphenylsulfonyloxy (Tosyloxy). Vorzugsweise bedeutet Y Hydroxy.

Halogenierungmittel, die geeignet sind, Y = z.B. Hydroxy in X = Fluor zu überführen, sind z.B. Fluorwasserstoff, Schwefeltetrafluorid, insbesondere ein Gemisch aus Fluorwasserstoff und Schwefeltetrafluorid und ferner auch z.B. substituierte Aminoschwefeltrifluoride, wie Diethylamino-schwefeltrifluorid (DAST) oder Piperidino-schwefeltrifluorid.

Halogenierungsmittel, die geeignet sind, Y = z.B. Hydroxy in X = Chlor, Brom oder lod zu überführen, sind z.B. die Halogenwasserstoffe, z.B. Chlor-, Brom- oder lodwasserstoff, Thionylhalogenide, z.B. Thionylchlorid oder -bromid, Sulfurylhalogenide, z.B. Sulfurylchlorid oder -bromid, oder Phosphorhalogenide, z.B. Phosphortribromid, -triiodid, -pentabromid, -pentaiodid, -trichlorid oder -pentachlorid.

Verbindungen der Formel II, worin Y Arylsulfonyloxy, z.B. Tosyloxy, bedeutet, können durch Umsetzung mit Alkalimetallhalogeniden, insbesondere Alkalimetallchloriden, -bromiden oder -iodiden, vorzugsweise in Dimethylformamid, in Verbindungen der Formel I, worin X Chlor, Brom oder lod bedeutet, umgewandelt werden.

Verbindungen der Formel II, worin Y Arylsulfonyloxy, z.B. Tosyloxy, bedeutet, können ferner z.B. durch Umsetzung mit $KHF_2$, etwa in 1,2-Dihydroxyethan, in Verbindungen der Formel I, worin X Fluor bedeutet, umgewandelt werden.

Insbesondere bei den letzten beiden Umsetzungen kann es erforderlich sein, die Aminogruppen in den Verbindungen der Formel II vor der Durchführung der Reaktion durch Aminoschutzgruppen, z.B. die unten genannten, zu schützen. Nach der Reaktion werden die Aminoschutzgruppen dann in an sich bekannter Weise wieder abgespalten.

Die Ausgangsverbindungen der Formel II, worin Y für Hydroxy steht, werden z.B. gemäss dem Verfahren (b), s.u., hergestellt. Die Ausgangsverbindungen der Formel II, worin Y aliphatisch- oder aromatisch-substituiertes Sulfonyloxy bedeuten, werden bevorzugt in an sich bekannter Weise aus Verbindungen der Formel II, worin Y für Hydroxy steht, hergestellt, z.B. durch Reaktion mit Niederalkylsulfonyl- oder Arylsulfonylchloriden.

Verfahren (b):

Die nucleophile Substitutionsreaktion gemäss Verfahren (b) wird ohne Lösungsmittel oder in Gegenwart eines Lösungsmittels, z.B. einem Niederalkanol oder Ether, wie Isopropanol oder Tetrahydrofuran, und gegebenenfalls unter erhöhtem Druck durchgeführt und erfolgt selektiv am terminalen C-Atom des Oxiranylrestes.

Die Ausgangsverbindungen der Formel III werden z.B. dadurch erhalten, dass man ein Hydroxylamin bzw. Oxim der Formel V mit z.B. Epichlorhydrin, Epibromhydrin oder 3-Tosyloxy-1,2-epoxypropan umsetzt. Diese Reaktion wird bevorzugt in Gegenwart einer Base, z.B. Natriumhydroxid, und ohne Lösungsmittel oder in Gegenwart eines Lösungsmittels, z.B. Aceton oder Acetonitril, durchgeführt.

Setzt man bei der Herstellung von Verbindungen der Formel III optisch aktives Epichlorhydrin, Epibromhydrin oder insbesondere 3-Tosyloxy-1,2-epoxypropan ein, so erhält man stereoselektiv die entsprechenden optisch aktiven Verbindungen der Formel III. Werden letztere beim Verfahren (b) eingesetzt, so gelangt man zu optisch aktiven Verbindungen der Formel I.

Die Verbindungen der Formel III können ferner auch dadurch erhalten werden, dass in Verbindungen der Formel VII, worin X Hydroxy bedeutet und W z.B. für Chlor steht, der Epoxyring in Gegenwart von Base geschlossen wird.

Verfahren (c):

Bevorzugte monovalente Aminoschutzgruppen S und S′ sind Estergruppen, z.B. Niederalkylester und insbesondere tert.-Butoxycarbonyl (BOC), oder Acylreste, z.B. Niederalkanoyl oder Halogenniederalkanoyl, wie insbesondere Acetyl, Chloracetyl oder Trifluoracetyl.

Bevorzugte bivalente Aminoschutzgruppen, gebildet aus den Resten S und $R_2$ bzw. S′ und $R_3$, sind mono- oder disubstituierte Methylidengruppen, wie 1-Niederalkoxy-niederalkylidengruppen, z.B. $=C(CH_3)-(OC_2H_5)$, ferner z.B. $=C(CH_3)_2$ oder =CH-Phenyl, die alle bevorzugt zum Schutz des Stickstoffatoms in der N-O-Gruppierung verwendet werden, und Bisacylreste, z.B. der von der Phthalsäure, welcher zusammen mit dem zu schützenden Stickstoffatom eine Phthalimidogruppe bildet.

Die Wirkungsweise von Schutzgruppen, z.B. Aminoschutzgruppen, ihre Einführung und Abspaltung sind an sich bekannt und z.B. in J.F.W. McOmie, "Protective Groups in Organic Chemistry", Plenum Press, London and New York 1973, und T.W. Greene, "Protective Groups in Organic Synthesis", Wiley, New York 1984 beschrieben.

Die Abspaltung der Aminoschutzgruppen kann z.B. hydrolytisch, insbesondere im sauren Medium, z.B. mit Chlorwasserstoff, verdünnter Schwefelsäure, Oxalsäure, organischen Sulfonsäuren, z.B. Toluol-4-sulfonsäure, oder Trifluoressigsäure, geschehen.

Bestimmte Aminoschutzgruppen, z.B. mono- oder disubstituierte Methylidengruppen und Niederalkanoyl, lassen sich durch Reduktion etwa mit komplexen Hydriden, z.B. $LiAlH_4$ oder $NaBH_3CN$, in eine Gruppe $R_1$ bzw. $R_3$ der Formel I überführen, z.B. $=C(CH_3)_2$ in Isopropyl oder Acetyl in Ethyl.

Verbindungen der Formel IV werden z.B. dadurch hergestellt, dass man eines der Verfahren (a), (b), (d), (e) oder (f) mit geschützter (geschützten) Aminogruppe(n) durchführt. Ferner können Verbindungen der Formel IV auch aus Verbindungen der Formel I - etwa zu Reinigungszwecken - hergestellt werden.

Verfahren (d):

In den Verbindungen der Formel VI ist die nucleofuge Abgangsgruppe Z genauso definiert wie eine nucleofuge Abgangsgruppe W in den Verbindungen der Formel VII unter Verfahren (e). Die Aminoschutzgruppen S in einer Verbindung der Formel VI entsprechen den in Verbindungen der Formel IV unter Verfahren (c) definierten Aminoschutzgruppe S bzw. S′.

Bedeutet in einer Verbindung der Formel VI Z Hydroxy, so kann man z.B. eine intermolekulare Dehydratisierungsreaktion durchführen. Insbesondere kommt dafür die Mitsunobu-Reaktion [s. Bull. Chem. Soc. Japan 40, 2380 (1967)] in Frage, bei der die Verbindungen der Formel V und VI mit z.B. Triphenylphosphin und N,N′-Azodicarbonsäureethylester umgesetzt werden.

Bedeutet in einer Verbindung der Formel VI Z eine nucleofuge Abgangsgruppe, so entspricht das Verfahren (d) einer einfachen nucleophilen Substitution (O-Alkylierung).

Bedeutet X in einer Verbindung der Formel VI Hydroxy, so kann es angezeigt sein, diese Hydroxygruppe vor der Durchführung des Verfahrens (d) durch eine Hydroxyschutzgruppe zu schützen, welche nach erfolgter Umsetzung gemäss Verfahren (d) wieder abgespalten wird. Geeignete Hydroxyschutzgruppen sind z.B. Acylderivate (Ester), wie etwa Niederalkanoyl, Benzoyl, Benzyloxycarbonyl oder Niederalkoxycarbonyl,

oder Alkylderivate (Ether), wie etwa Niederalkoxyniederalkyl, 2-Tetrahydropyranyl, Trityl oder Benzyl, ferner auch Triniederalkylsilyl.

Bedeutet X in einer Verbindung der Formel VI Hydroxy, so kann diese Hydroxygruppe auch gemeinsam mit der Aminogruppe -$NSR_3$ geschützt werden. Dies kann z.B. dadurch geschehen, dass die Reste X und S zusammen in einer Verbindung der Formel VI eine Gruppe -O-$CH_2$- bilden, wobei ein Oxazolidinring gebildet wird. Eine derart geschützte Verbindung wird z.B. aus der freien Hydroxyaminoverbindung durch Kondensation mit Formaldehyd in an sich bekannter Weise erhalten. Nach der Durchführung des Verfahrens (d) wird diese Schutzgruppe im sauren Medium wieder abgespalten.

Verbindungen der Formel VI, worin X und Z Hydroxy bedeuten, werden z.B. dadurch hergestellt, dass man 2,3-Epoxypropanol mit Ammoniak bzw. einem Amin der Formel $NH_2R_3$ umsetzt und dann gegebenenfalls eine Hydroxy-und/oder Aminoschutzgruppe einführt.

Verfahren (e):

In einer Verbindung der Formel VII bedeutet ein in Amino überführbarer Rest W z.B. eine Azidogruppe (-$N_3$), welche durch Reduktion, etwa mit komplexen Metallhydriden, z.B. $LiAlH_4$, Zinn(II)-chlorid oder mit Wasserstoff in Gegenwart eines Katalysators, z.B. Palladium auf Kohle, in Amino überführt werden kann. Es kann angezeigt sein, vor der Durchführung des Verfahrens (e) in der Verbindung der Formel VII eine Hydroxygruppe X und/oder die Aminogruppe $R_1R_2N$- mit einer Hydroxy- bzw. Aminoschutzgruppe zu schützen. Solche Schutzgruppen werden nach der Durchführung des Verfahrens (e) wieder abgespalten.

Verbindungen der Formel VII, worin in Azido bedeutet, können z.B. durch die Umsetzung von Verbindungen der Formel III oder IIIa mit z.B. Natrium-oder Ammoniumazid erhalten werden.

Der Rest W in einer Verbindung der Formel VII kann ferner z.B. eine nucleofuge Abgangsgruppe bedeuten. Eine solche nucleofuge Abgangsgruppe W ist bevorzugt Halogen, z.B. Chlor, Brom oder Iod, ferner aber auch z.B. aliphatisch- oder aromatisch-substituiertes Sulfonyloxy, z.B. Methylsulfonyloxy oder 4-Methylphenylsulfonyloxy.

Eine derartige Verbindung der Formel VII lässt sich durch Umsetzung mit Ammoniak oder einem Amin der Formel $NH_2R_3$ im Sinne einer nucleophilen Substitutionsreaktion in eine Verbindung der Formel I überführen.

Ausgangsverbindungen der Formel VII werden z.B. aus Verbindungen der Formel III oder aus Verbindungen der Formel IX

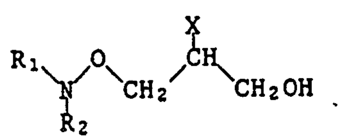

(IX)

durch Umsetzung mit den entsprechenden Nucleophilen, z.B. Halogenid, Mesylat oder Tosylat, hergestellt.

Verbindungen der Formel VII, worin X für Hydroxy steht und W z.B. Halogen oder Tosyloxy bedeutet, können ferner durch Umsetzung von Verbindungen der Formel V mit Epihalogenhydrin oder 3-Tosyloxy-1,2-epoxypropan, insbesondere im sauren Medium, erhalten werden.

Verbindungen der Formel IX, worin X Hydroxy bedeutet, können analog aus Verbindungen der Formel V durch Umsetzung mit 2,3-Epoxypropanol erhalten werden.

Verbindungen der Formel IX können weiterhin z.B. auch ausgehend von Verbindungen der Formel V mit z.B. 3-Halogen-2-X-propanolen, worin gegebenenfalls die Hydroxygruppe geschützt vorliegt, erhalten werden.

Verfahren (f):

Die Reduktion kann z.B. mit den gleichen Reduktionsmitteln durchgeführt werden, die beim Verfahren (e) für die Reduktion von Azidogruppen angegeben sind.

Die Ausgangsverbindungen der Formel VIII lassen sich z.B. aus den analogen Aldehyden (=O statt =$NR_3$ in Formel VIII) durch Umsetzung mit Ammoniak, Hydroxylamin bzw. Niederalkylaminen herstellen.

Die erwähnten Aldehyde analog zur Formel VIII lassen sich z.B. durch Oxidation, z.B. mit Pyridiniumchlorchromat [vgl. J. Org. Chem. 46, 4797 (1981)], aus entsprechenden Hydroxymethylverbindungen

herstellen. Weiterhin sind sie auch durch Reduktion von entsprechenden Niederalkylestern, z.B. mit Diisobutylaluminiumhydrid [vgl. Chem. Pharm. Bull. 23, 3081 (1975)], oder durch Reduktion von entsprechenden Säurechloriden, z.B. mit Tri-n-butylzinnhydrid [vgl. J. Org. Chem. 25, 284 (1961) oder J. Amer. Chem. Soc. 88, 571 (1966)] erhältlich.

Die erwähnten Aldehyde analog zur Formel VIII können insbesondere auch über die folgende Reaktionssequenz erhalten werden:

(1) Umsetzung von 3,4-0,0-Isopropyliden-3,4-dihydroxy-1,2-epoxybutan [s. J. Org. Chem. 52, 2841 (1987) oder DE-A-3,150,917] mit Acethydroxamsäureethylester zum 3,4-0,0-Isopropyliden-2,3,4-trihydroxy-1-(1-ethoxyethylidenaminoxy)-butan. In letzterem kann die 2-Hydroxygruppe gegebenenfalls in an sich bekannter Weise in einen anderen Rest X der Formel I, z.B. Halogen, umgewandelt oder durch eine Schutzgruppe geschützt werden.

(2) Abspaltung der Isopropylidengruppe z.B. durch Behandlung mit verdünnter Säure.

(3) Glykolspaltung der endständigen a,$\beta$-Dihydroxyethylgruppe zu Formyl durch Umsetzung mit $NaIO_4$ oder $Pb(OCOCH_3)_4$.

(4) Abspaltung der 1-Ethoxyethylidengruppe z.B. durch Behandlung mit Säure.

Verbindungen der Formel I können in andere Verbindungen der Formel I umgewandelt werden.

Verbindungen der Formel I, worin X für Hydroxy steht, können in an sich bekannter Weise in Verbindungen der Formel I, worin X Niederalkoxy, Acyloxy, Niederalkylsulfonyloxy oder Arylsulfonyloxy bedeutet, umgewandelt werden, indem man erstere mit Niederalkylierungsmitteln, z.B. Niederalkylhalogeniden, -tosylaten oder -mesylaten oder Diniederalkylsulfaten, bzw. Acylierungsmitteln, z.B. Säureanhydriden oder -halogeniden, bzw. Niederalkylsulfonylhalogeniden bzw. Arylsulfonylhalogeniden, z.B. 4-Methylphenylsulfonylchlorid, umsetzt. Bevorzugt sind bei diesen Umsetzungen die beiden Aminogruppen in den Verbindungen der Formel I geschützt.

Bei der Durchführung einer oben erwähnten Alkylierung von X = OH in X = Niederalkoxy kann gleichzeitig auch die Aminogruppe $-NR_1R_2$ ($R_1$ und $R_2$ stehen für Wasserstoff) niederalkyliert werden, selbst wenn sie durch eine Aminoschutzgruppe, z.B. BOC, geschützt ist.

Verbindungen der Formel I, worin $R_1$ und $R_2$ zusammen für einen Rest $R_5R_4C=$ stehen, lassen sich durch Reduktion, z.B. mit Natriumcyanoborhydrid, in andere Verbindungen der Formel I, worin $R_1$ Wasserstoff bedeutet und $R_2$ für einen Rest $-CH_4R_5$ steht, überführen.

Verbindungen der Formel I, worin $R_1$ und $R_2$ Wasserstoff bedeuten, lassen sich durch Umsetzung mit einer Carbonylverbindung $R_5R_4C=O$, worin die Carbonylgruppe auch maskiert vorliegen kann - etwa als Acetal oder Ketal -, in andere Verbindungen der Formel I überführen, worin die Reste $R_1$ und $R_2$ zusammen für eine Gruppe $R_5R_4C=$ stehen.

Umgekehrt können auch Verbindungen der Formel I, worin $R_1$ und $R_2$ zusammen für eine Gruppe $R_5R_4C=$ stehen, z.B. durch saure Hydrolyse in Verbindungen der Formel I umgewandelt werden, worin $R_1$ und $R_2$ Wasserstoff bedeuten.

Verfahrensgemäss erhältliche freie Verbindungen der Formel I mit salzbildenden Eigenschaften können in an sich bekannter Weise in ihre Salze übergeführt werden, Verbindungen mit basischen Eigenschaften durch Behandeln mit Säuren oder geeigneten Derivaten davon.

Die Verbindungen, einschliesslich ihrer Salze, können auch in Form ihrer Hydrate erhalten werden, oder ihre Kristalle können z.B. das zur Kristallisation verwendete Lösungsmittel einschliessen.

Erfindungsgemäss erhältliche Gemische von Isomeren können in an sich bekannter Weise in die einzelnen Isomeren aufgetrennt werden, Racemate z.B. durch Bilden von Salzen mit optisch reinen salzbildenden Reagentien und Auftrennen des so erhältlichen Diastereomerengemisches, z.B. mittels fraktionierter Kristallisation.

Die oben angeführten Reaktionen können unter an sich bekannten Reaktionsbedingungen durchgeführt werden, in Ab- oder üblicherweise Anwesenheit von Lösungs- oder Verdünnungsmitteln, vorzugsweise solchen, die gegenüber den verwendeten Reagenzien inert sind und diese lösen, in Ab- oder Anwesenheit von Katalysatoren, Kondensationsmitteln oder neutralisierenden Agentien, je nach Art der Reaktion und/oder der Reaktionsteilnehmer bei erniedrigter, normaler oder erhöhter Temperatur, z.B. im Temperaturbereich von etwa -80°C bis etwa 190°C, vorzugsweise von etwa -20°C bis etwa 150°C, z.B. beim Siedepunkt des verwendeten Lösungsmittels, unter atmosphärischem Druck oder in einem geschlossenen Gefäss, gegebenenfalls unter Druck, und/oder in einer inerten Atmosphäre, z.B. unter einer Stickstoffatmosphäre.

Im Verfahren der vorliegenden Erfindung werden vorzugsweise solche Ausgangsstoffe eingesetzt, die zu den eingangs als besonders wertvoll beschriebenen Verbindungen führen.

Die Erfindung betrifft auch diejenigen Ausführungsformen des Verfahrens, bei denen man von einer auf einer beliebigen Verfahrensstufe als Zwischenprodukt erhältlichen Verbindung ausgeht und die fehlenden Verfahrensschritte durchführt, oder bei denen ein Ausgangsstoff unter den Reaktionsbedingungen gebildet

oder in Form eines Derivates, z.B. eines Salzes davon, verwendet wird.

Die vorliegende Erfindung betrifft ebenfalls pharmazeutische Präparate, die als Wirkstoff eine der pharmakologisch wirksamen Verbindungen der Formel I enthalten. Besonders bevorzugt sind Präparate zur enteralen, insbesondere oralen, sowie zur parenteralen Verabreichung. Die Präparate enthalten den Wirkstoff allein oder vorzugsweise zusammen mit einem pharmazeutisch anwendbaren Trägermaterial. Die Dosierung des Wirkstoffs hängt von der zu behandelnden Krankheit, sowie von Spezies, deren Alter, Gewicht und individuellem Zustand, sowie von der Applikationsweise ab.

Die pharmazeutischen Präparate enthalten von etwa 5 % bis etwa 95 % des Wirkstoffs, wobei einzeldosierte Applikationsformen vorzugsweise von etwa 20 % bis etwa 90 % und nicht-einzeldosierte Applikationsformen vorzugsweise etwa 5 % bis etwa 20 % Wirkstoff aufweisen. Dosiseinheitsformen, wie Dragées, Tabletten oder Kapseln, enthalten von etwa 0,05 g bis etwa 1,0 g des Wirkstoffs.

Die pharmazeutischen Präparate der vorliegenden Erfindung werden in an sich bekannter Weise, z.B. mittels konventioneller Misch-, Granulier-, Dragier-, Lösungs-, oder Lyophilisierungsverfahren hergestellt. So kann man pharmazeutische Zusammensetzungen zur oralen Anwendung erhalten, indem man den Wirkstoff mit einem oder mehreren festen Trägerstoffen kombiniert, ein erhaltenes Gemisch gegebenenfalls granuliert, und das Gemisch bzw. Granulat, wenn erwünscht, gegebenenfalls durch Zugabe von zusätzlichen Hilfsstoffen, zu Tabletten oder Dragées-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärken, z.B. Mais-, Weizen-, Reis- oder Kartoffelstärke, Methylcellulose, Hydroxypropylmethylcellulose, Natriumcarboxymethylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Alginsäure oder ein Salz davon, wie Natriumalginat. Zusätzliche Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol, oder Derivate davon.

Dragées-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Oral anwendbare pharmazeutische Zusammensetzungen sind ebenfalls Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Maisstärke, Bindemitteln und/oder Gleitmitteln, wie Talk oder Magnesiumstearat, und gegebenenfalls von Stabilisatoren, enthalten. In weichen Kapseln ist der Wirkstoff vorzugsweise in geeigneten flüssigen Hilfsstoffen, wie fetten Oelen, Paraffinöl oder flüssigen Polyethylenglykolen, gelöst oder suspendiert, wobei ebenfalls Stabilisatoren zugefügt sein können.

Weitere orale Applikationsformen sind z.B. in üblicher Weise bereitete Sirups, die den Wirkstoff z.B. in suspendierter Form und in einer Konzentration von ca. 5 % bis 20 %, vorzugsweise ca. 10 % oder in einer ähnlichen Konzentration, die z.B. beim Abmessen von 5 oder 10 ml eine geeignete Einzeldosis ergibt, enthalten. Ferner kommen z.B. auch pulverförmige oder flüssige Konzentrate zur Bereitung von Shakes, z.B. in Milch, in Betracht. Solche Konzentrate können auch in Einzeldosismengen abgepackt sein.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole.

Zu parenteralen Verabreichung eignen sich in erster Linie wässrige Lösungen eines Wirkstoffes in wasserlöslicher Form, z.B. eines wasserlöslichen Salzes, oder wässrige Injektionssuspensionen, welche viskositätserhöhende Stoffe, z.B. Natriumcarboxymethylcellulose, Sorbit und/oder Dextran und gegebenenfalls Stabilisatoren enthalten. Dabei kann der Wirkstoff, gegebenenfalls zusammen mit Hilfsstoffen, auch in Form eines Lyophilisats vorliegen und vor der parenteralen Verabreichung durch Zugabe von geeigneten Lösungsmitteln in Lösung gebracht werden.

Lösungen, wie sie z.B. für die parenterale Verabreichung verwendet werden, können auch als Infusionslösungen angewandt werden.

Die Erfindung betrifft ebenfalls ein Verfahren zur Behandlung der oben genannten Krankheitszustände. Die Verbindungen der vorliegenden Erfindung können prophylaktisch oder therapeutisch verabreicht werden, wobei man sie vorzugsweise in Form von pharmazeutischen Präparaten verwendet. Dabei wird bei einem Körpergewicht von etwa 70 kg eine tägliche Dosis von etwa 0,3 g bis etwa 15 g, vorzugsweise von etwa 0,5 g bis etwa 5 g einer Verbindung der vorliegenden Erfindung verabreicht.

Die nachfolgenden Beispiele illustrieren die vorliegende Erfindung; Temperaturen werden in Grad Celsius angegeben. Folgende Abkürzugen werden verwendet: Essigester ≙ Essigsäureethylester; BOC ≙, tert.-Butyloxycarbonyl; THF ≙ Tetrahydrofuran; Hexan ≙ n-Hexan; Ether ≙ Diethylether; Mesyl ≙ Methylsulfonyl.

Beispiel 1:

In einem goldbeschichteten 50 ml Autoklav werden 3,2 g N-(3-Aminoxy-2-hydroxypropyl)-isopropylamin bei -78° in 20 g wasserfreiem Fluorwasserstoff gelöst und mit 5,4 g Schwefeltetrafluorid versetzt. Der Autoklav wird verschlossen, 3 h bei -78° mit einem Magnetstab gerührt, auf 0° erwärmt und nach weiteren 24 h entgast. Der Rückstand wird in 30 ml 2N Salzsäure aufgenommen, filtriert und auf eine 35 x 270 mm Säule mit schwach basischem Ionenaustauscher MWA-1 (Dow Chemicals) aufgetragen. Die Säule wird mit Wasser gewaschen. Die Ninhydrin-positiven Fraktionen werden vereinigt, mit Salzsäure neutralisiert und eingedampft. Der hygroskopische Rückstand entspricht N-(3-Aminoxy-2-fluorpropyl)-isopropylamin-dihydrochlorid; er wird aus Essigester kristallisiert und über Phosphorpentoxid getrocknet; Rf-Wert = 0,55 (Kieselgel/Methylenchlorid:Methanol:konz. Ammoniak 150:50:1).

Das als Ausgangsmaterial verwendete N-(3-Aminoxy-2-hydroxypropyl)-isopropylamin wird aus dem entsprechenden Dihydrochlorid (s. DE-C-2 651 083) durch Filtration über einen stark basischen Ionenaustauscher, z.B. die 50-fache Menge von DOWEX 1x4 (OH), freigesetzt.

Beispiel 2:

Eine Lösung von 3 g N,N′-Di-BOC-N-(3-aminoxy-2-fluorpropyl)-isopropylamin in 30 ml Essigester wird mit 30 ml einer ca. 2N Lösung von Chlorwasserstoff in Essigester versetzt und unter Feuchtigkeitsauschluss 18 h bei Raumtemperatur gerührt. Das ausgefallene Produkt wird abgesaugt, mit Essigester und Ether gewaschen und getrocknet. Man erhält so das N-(3-Aminoxy-2-fluorpropyl)-isopropylamin-dihydrochlorid als weisses hygroskopisches Pulver, Rf-Wert = 0,55 (Kieselgel/Methylenchlorid:Methanol:konz. Ammoniak 150:50:1).

Das Ausgangsmaterial wird wie folgt hergestellt:

Das Rohprodukt aus dem in Beispiel 1 beschriebenen Fluorierungsansatz, ausgehend von 20 mMol N-(3-Aminoxy-2-fluorpropyl)-isopropylamin, wird in 30 ml 2N Salzsäure gelöst. Diese Lösung wird mit 50 ml Wasser und 50 ml THF verdünnt und portionsweise mit festem Natriumhydrogencarbonat neutralisiert. Dieses Gemisch wird anschliessend unter Eiskühlung mit einer Lösung von 12 g Di-tert.-butyldicarbonat (Fluka) in 50 ml THF tropfenweise versetzt und 20 h bei Raumtemperatur gerührt. Das Reaktionsgemisch wird mit 250 ml Ether verdünnt. Die obere Schicht wird mit Wasser und einer gesättigten Kochsalzlösung gewaschen, über Magnesiumsulfat getrocknet und eingedampft. Das zurückgebliebene Oel wird mit einem Gemisch Hexan/Essigester (2:1) über Kieselgel chromatographiert, und man erhält das N,N′-Di-BOC-N-(3-aminoxy-2-fluorpropyl)-isopropylamin als farbloses Oel, Rf-Wert = 0,32 (Kieselgel/Hexan:Essigester 2:1).

Beispiel 3:

Eine Lösung von 360 mg (2 mMol) 3-Aminoxy-2-hydroxypropylamin-dihydrochlorid in 5 ml Ethanol wird mit 2 ml 1N Natronlauge und 0,2 ml (2 mMol) Benzaldehyd versetzt und 16 h bei Raumtemperatur gerührt. Das Reaktionsgemisch wird eingedampft, mit 10 ml Wasser und 1 ml 2N Natronlauge versetzt und zweimal mit 50 ml Ether extrahiert. Die Etherphasen werden mit verdünnter Kochsalzlösung gewaschen, vereinigt, filtriert und eingedampft. Der Rückstand wird aus wenig Ether kristallisiert und entspricht 3-Benzylidenaminoxy-2-hydroxypropylamin, Smp. 65°, Rf-Wert = 0,32 (Kieselgel/Methylenchlorid:Methanol:konz. Ammoniak 40:10:1).

Beispiel 3a:

Eine Lösung von 1,77 g (10 mMol) 1-Benzylidenaminoxy-2,3-epoxy-propan [s. z.B. Zh. Org. Khimii 5, 1353 (1969)] in 10 ml THF wird mit 40 ml konz. Ammoniak versetzt und 16 h bei Raumtemperatur gerührt. Das Reaktionsgemisch wird eingedampft und in 100 ml Ether aufgenommen. Diese Lösung wird über Natriumsulfat getrocknet, filtriert und eingeengt, wobei das 3-Benzylidenaminoxy-2-hydroxypropylamin nach dem Abkühlen auskristallisiert, Smp. 65°.

Beispiel 4:

Analog dem in Beispiel 3 beschriebenen Verfahren wird 3-Aminoxy-2-hydroxypropylamin-dihydrochlorid mit Cyclopentanon umgesetzt. Man erhält das 3-Cyclopentylidenaminoxy-2-hydroxypropylamin als Wachs, Rf-Wert = 0,33 (Kieselgel/Methylenchlorid:Methanol:konz. Ammoniak 40:10:1).

Beispiel 4a:

Analog Beispiel 3a wird 1-Cyclopentylidenaminoxy-2,3-epoxypropan [s. J. Med. Chem. 28, 153 (1985)] mit konz. Ammoniak umgesetzt und aufgearbeitet. Das Rohprodukt wird mit dem System Methylenchlorid/Methanol/konz. Ammoniak (40:10:1) chromatographiert, wobei man das 3-Cyclopentylidenaminoxy-2-hydroxypropylamin als Wachs erhält, Rf-Wert = 0,33 (Kieselgel/Methylenchlorid:Methanol:konz. Ammoniak 40:10:1).

Beispiel 5:

In einem 200 ml Teflon-Reaktor werden 2,5 g 3-Aminoxy-2-hydroxypropylamin (s. DE-C-2 651 083) bei -78° in 40 g flüssigem Fluorwasserstoff (HF) gelöst. Anschliessend werden noch 5,6 g Schwefeltetrafluorid eingeleitet. Das Gemisch im Reaktor wird verschlossen 3 h bei -78° mit einem Magnetstab gerührt, auf 0° erwärmt und nach weiteren 24 h entgast. Man erhält so das rohe 3-Aminoxy-2-fluorpropylamin als Hydrofluorid, das mit einer der beiden folgenden Methoden in das entsprechende reine Dihydrochlorid überführt wird:

a) Der Rückstand wird in 50 ml 2N HCl aufgenommen, filtriert und auf eine 35 x 270 mm Säule mit schwach basischem Ionenaustauscher MWA-1 (Dow·Chemicals) aufgetragen. Die Säule wird mit Wasser gewaschen. Die Ninhydrin-positiven Fraktionen werden vereinigt, mit 1N HCl neutralisiert und eingedampft. Der Rückstand wird aus Essigester kristallisiert, und man erhält 3-Aminoxy-2-fluorpropylamin-dihydrochlorid vom Smp. 204-207°.

b) Der Rückstand wird in 50 ml 2N HCl und 100 ml Wasser aufgenommen, filtriert und mit 100 ml THF verdünnt. Dieses Gemisch wird unter Eiskühlung portionsweise mit festem Natriumhydrogencarbonat bis pH 7,5 neutralisiert und dann mit einer Lösung von 13,0 g Di-tert.-butyl-dicarbonat (Fluka) in 70 ml THF tropfenweise versetzt. Das zweiphasige Reaktionsgemisch wird 16 h bei Raumtemperatur gerührt und anschliessend mit 100 ml Ether verdünnt. Die organische Phase wird abgetrennt, mit 100 ml Wasser und 50 ml einer gesättigten Kochsalzlösung gewaschen, über Magnesiumsulfat getrocknet und nach der Abtrennung des Trocknungsmittels eingedampft. Der Rückstand wird aus Ether und Hexan kristallisiert und entspricht N,N′-Di-BOC-3-aminoxy-2-fluorpropylamin vom Smp. 67-69°.

Eine Lösung von 1,8 g N,N′-Di-BOC-3-aminoxy-2-fluorpropylamin in 10 ml Essigester wird mit 20 ml einer 1,8N Lösung von Chlorwasserstoff in Essigester versetzt und während 18 h bei Raumtemperatur gerührt. Das auskristallisierte Produkt wird abgesaugt, mit Ether gewaschen und getrocknet. Es entspricht 3-Aminoxy-2-fluorpropylamin-dihydrochlorid vom Smp. 204-207°.

Beispiel 6:

Analog Beispiel 2 werden 3,4 g N,N′-Di-BOC-3-aminoxy2-mesyloxy-propylamin mit 30 ml einer ca. 1,5 N Lösung von Chlorwasserstoff in Essigester behandelt und aufgearbeitet. Man erhält so das 3-Aminoxy-2-methylsulfonyloxy-propylamin-dihydrochlorid als weisse hygroskopische Kristalle, Rf-Wert = 0,37 (Kieselgel/Methylenchlorid:Methanol:konz. Ammoniak 40:10:1).

Das Ausgangsmaterial wird wie folgt hergestellt:

Eine Lösung von 18 g (0,1 Mol) 3-Aminoxy-2-hydroxy-propylamin-dihydrochlorid und 21,2 g (0,2 Mol)

wasserfreiem Natriumcarbonat in 100 ml Wasser und 100 ml THF wird unter Eiswasserkühlung und Rühren tropfenweise mit einer Lösung von 48 g (0,22 Mol) Di-tert.-butyl-dicarbonat (Fluka) in 100 ml THF versetzt und 4 h bei Raumtemperatur gerührt. Das Reaktionsgemisch wird mit 250 ml Ether verdünnt. Man trennt die obere Schicht ab, wäscht diese mit Wasser und einer gesättigten Kochsalzlösung, trocknet über Magnesiumsulfat und dampft ein. Das zurückgebliebene N,N′-Di-BOC-3-aminoxy-2-hydroxy-propylamin wird aus Ether/Petrolether kristallisiert, Smp. 106-108°.

Eine Lösung von 2,75 g (9 mMol) N,N′-Di-BOC-3-aminoxy-2-hydroxy-propylamin in 10 ml Pyridin wird unter Rühren, Eiswasserkühlung und Feuchtigkeitsausschluss mit 1,26 g (11 mMol) Methansulfochlorid versetzt und 16 h bei Raumtemperatur gerührt. Das Reaktionsgemisch wird anschliessend mit 2 ml Wasser versetzt und eingedampft. Der Rückstand wird mit 100 ml Ether und 50 ml Wasser versetzt. Man trennt die ober Schicht ab, wäscht diese nacheinander mit einer verdünnten Natriumhydrogensulfatlösung, Wasser, einer gesättigten Natriumhydrogencarbonatlösung und wieder mit Wasser und dampft nach dem Trocknen über Magnesiumsulfat ein. Man erhält so das N,N′-Di-BOC-3-aminoxy-2-mesyloxy-propylamin als gelbes Oel, Rf-Wert = 0,28 (Kieselgel/Hexan:Essigester 1:1).

Beispiel 7:

Analog Beispiel 2 werden 1,3 g N,N′-Di-BOC-3-methylaminoxy-2-methoxy-propylamin mit 20 ml einer ca. 1,5 N Lösung von Chlorwasserstoff in Essigester behandelt und aufgearbeitet. Das Rohprodukt wird in wenig Wasser gelöst und über 100 ml DOWEX 1x4 ($OH^{\ominus}$ Form) filtriert. Das Filtrat wird eingedampft und der Rückstand aus 10 ml Methanol unter Zusatz von 700 mg Oxalsäure kristallisiert. Man erhält so das 3-Methylaminoxy-2-methoxy-propylamin-dioxalat, Smp. 153-155°.

Das Ausgangsmaterial wird wie folgt hergestellt:

Eine Lösung von 3,06 g (10 mMol) N,N′-Di-BOC-3-aminoxy-2-hydroxy-propylamin in 100 ml THF wird unter Rühren und Feuchtigkeitsausschluss mit 1,7 g (40 mMol) einer 55-60%igen NaH-Dispersion in Oel versetzt. Man gibt noch eine Lösung von 1,9 ml (20 mMol) Dimethylsulfat in 10 ml THF tropfenweise zu und rührt 15 h bei Raumtemperatur nach. Das Reaktionsgemisch wird eingedampft, der Rückstand in Ether aufgenommen, mit Wasser gewaschen, über Magnesiumsulfat getrocknet und wieder eingedampft. Das zurückgebliebene Oel wird mt einem Gemisch Hexan/Essigester (1:1) über Kieselgel chromatographiert, und man erhält das N,N′-Di-BOC-3-methylaminoxy-2-methoxy-propylamin als blassgelbes Oel, Rf-Wert = 0,36 (Kieselgel/Hexan:Essigester 1:1).

Beispiel 8:

Analog Beispiel 3 wird 3-Aminoxy-2-hydroxypropylamin-dihydrochlorid mit Salicylaldehyd umgesetzt. Man erhält das 3-Salicylidenaminoxy-2-hydroxypropylamin [≙ 3-(2-Hydroxybenzylidenaminoxy)-2-hydroxypropylamin] als gelbe Kristalle vom Smp. 94-97°.

Beispiel 9:

Analog Beispiel 3 wird 3-Aminoxy-2-hydroxypropylamin-dihydrochlorid mit Aceton umgesetzt. Man erhält das 3-Isopropylidenaminoxy-2-hydroxypropylamin, das als Hydrochlorid aus Isopropanol/Ether kristallisiert wird. Die weissen hygroskopischen Kristalle werden über Phosphorpentoxid getrocknet, Rf-Wert = 0,1 (Kieselgel/Methylenchlorid:Methanol:konz. Ammoniak 150:50:1).

Beispiel 9a:

Analog Beispiel 3a wird 1-Isopropylidenaminoxy-2,3-epoxypropan [siehe Zh. Org. Khimii 5, 1353 (1969)] mit konz. Ammoniak umgesetzt. Das Reaktionsgemisch wird zur Trockene eingedampft. Man erhält das 3-Isopropylidenaminoxy-2-hydroxypropylamin als blassgelbes Oel, Rf-Wert = 0,1 (Kiesegel/Methylenchlorid:Methanol:konz. Ammoniak 150:50:1).

Beispiel 10:

Analog Beispiel 2 werden 4,5 g N-BOC-(3-isopropylaminoxy-2-hydroxypropyl)-amin mit 50 ml einer ca. 1,5 N Lösung von Chlorwasserstoff in Essigester behandelt. Das Reaktionsgemisch wird eingedampft, das Rohprodukt in wenig Wasser gelöst und über 200 ml DOWEX 1x4 ($OH^{\ominus}$ Form) filtriert. Das Filtrat wird eingedampft, der ölige Rückstand in Essigester aufgenommen, über Natriumsulfat getrocknet und wieder vom Lösungsmittel befreit. Das langsam durchkristallisierende 3-Isopropylaminoxy-2-hydroxypropylamin wird bei 50°/0,1 mbar sublimiert.

Das Ausgangsmaterial wird wie folgt hergestellt:

Eine Lösung von 13,48 g 3-Isopropylidenaminoxy-2-hydroxypropylamin in je 150 ml THF und Wasser wird unter Rühren und Eiswasserkühlung mit einer Lösung von 28 g Di-tert.-butyl-dicarbonat (Fluka) in 150 ml THF tropfenweise versetzt und 16 h bei Raumtemperatur gerührt. Das Reaktionsgemisch wird im Vakuum vom THF befreit und mit Ether extrahiert. Die organische Phase wird mit Wasser und gesättigter Kochsalzlösung gewaschen, über Magnesiumsulfat getrocknet und eingedampft. Der Rückstand wird mit einem Gemisch Hexan/Essigester (1:1) über Kieselgel chromatographiert, und man erhält das N-BOC-3-isopropylidenaminoxy-2-hydroxypropylamin als blassgelbes Oel, Rf-Wert = 0,16 (Kieselgel/Hexan:Essigester 1:1).

Eine Lösung von 10,32 g (41,95 mMol) N-BOC-3-isopropylidenaminoxy-2-hydroxypropylamin in 230 ml THF wird mit 17,3 ml (304 mMol) Eisessig und 9,6 g (152 mMol) Natriumcyanoborhydrid versetzt und 16 h bei Raumtemperatur gerührt. Das Reaktionsgemisch wird mit 10,32 g Natriumhydrogencarbonat versetzt, 30 min nachgerührt und eingedampft. Der Rückstand wird mit Ether aufgenommen, mit Wasser gewaschen, über Magnesiumsulfat getrocknet und vom Lösungsmittel befreit. Das Produkt wird mit einem Gemisch Hexan/Essigester (1:1) über Kieselgel chromatographiert, und man erhält das N-BOC-(3-isopropylaminoxy-2-hydroxypropyl)-amin als blassgelbes Oel, Rf-Wert = 0,05 (Kieselgel/Hexan:Essigester 1:1).

Beispiel 11:

Eine Lösung von 2,6 g N-BOC-(3-isopropylaminoxy-2-fluorpropyl)-amin in 25 ml Ethanol wird mit 10 ml einer ca. 5,5 N Lösung von HCl in Ethanol versetzt, unter Feuchtigkeitsausschluss 18 h bei Raumtemperatur stehengelassen und zur Trockene eingedampft. Der Rückstand wird in Wasser gelöst und einmal mit Ether extrahiert. Die Wasserphase wird mit Aktivkohle filtriert und lyophilisiert. Man erhält so das 3-Isopropylaminoxy-2-fluorpropylamin-dihydrochlorid als gelbliches hygroskopisches Pulver, Rf-Wert = 0,59 (Kieselgel/Methylenchlorid:Methanol:konz. Ammoniak 40:10:1).

Das Ausgangsmaterial wird wie folgt hergestellt:

Analog Beispiel 5 werden 2,65 g (17,9 mMol) 3-Isopropylaminoxy-2-hydroxypropylamin in 20 ml wasserfreiem Fluorwasserstoff mit 4,2 g (40 mMol) Schwefeltetrafluorid fluoriert und mit 10 g Di-tert.-butyl-dicarbonat (Fluka) in ein entsprechendes (Mono-)BOC-Derivat umgewandelt. Das Rohprodukt wird mit einem Gemisch Hexan/Essigester (2:1) über Kieselgel chromatographiert, und man erhält das N-BOC-(3-isopropylaminoxy-2-fluorpropyl)-amin als blassgelbes Oel, Rf-Wert = 0,12 (Kieselgel/Hexan:Essigester 2:1).

Beispiel 12:

Analog Beispiel 3 werden 537 mg (3 mMol) 3-Aminoxy-2-hydroxypropylamin-dihydrochlorid in 10 ml Ethanol und 3 ml 1 N Natronlauge mit 0,61 g (3 mMol) Pyridoxal-hydrochlorid umgesetzt. Das Reaktionsgemisch wird eingedampft und der kristalline Rückstand aus Methanol umkristallisiert. Man erhält so das 3-Pyridoxalaminoxy-2-hydroxypropylamin-dihydrochlorid als weisse Kristalle, Smp. >185° (Zersetzung), Rf-Wert = 0,11 (Kieselgel/Methylenchlorid:Methanol:konz. Ammoniak 40:10:1).

Beispiel 13:

Eine Lösung von 450 mg (2,5 mMol) 3-Aminoxy-2-fluorpropylamin-dihydrochlorid (s. Beispiel 5) in 5 ml Ethanol und 2,5 ml 1N Natronlauge wird mit 0,14 ml Acetaldehyd versetzt und 16 Stunden bei Raumtemperatur gerührt. Das Reaktionsgemisch wird eingedampft und der Rückstand zwischen Wasser und Ether verteilt. Die untere Phase wird eingeengt und lyophilisiert. Man erhält das 3-Ethylidenaminoxy-2-fluor propylamin-hydrochlorid als weisses Pulver, das 1 Mol Kochsalz enthält, Rf-Wert 0,27 (Kieselgel/Methylenchlorid:Methanol:konz. Ammoniak 300:50:1).

Beispiel 14:

3-Ethylidenaminoxy-2-fluorpropylamin (s. Beispiel 13) wird mit Natriumcyanoborhydrid in analoger Weise wie in Beispiel 10 beschrieben zu 3-Ethylaminoxy-2-fluorpropylamin reduziert, Rf-Wert 0,2 (Kieselgel/Methylenchlorid:Methanol:konz. Ammoniak 40:10:1).

Beispiel 15:

Eine Lösung von 900 mg (5,0 mMol) 3-Aminoxy-2-fluorpropylamin-dihydrochlorid (s. Beispiel 5) in 10 ml Ethanol und 5,0 ml 1N Natronlauge wird mit 0,44 ml (6 mMol) Aceton versetzt und 16 Stunden bei Raumtemperatur gerührt. Das Reaktionsgemisch wird eingedampft und der Rückstand zwischen Wasser und Ether verteilt. Die untere Phase wird eingeengt und lyophilisiert. Man erhält das 3-Isopropylidenaminoxy-2-fluor-propylamin-hydrochlorid als weisses Pulver, das 1 Mol Kochsalz enthält, Smp. 90°, Rf-Wert 0,30 (Kieselgel/Methylenchlorid:Methanol:konz. Ammoniak 300:50:1).

Beispiel 16:

3-Isopropylidenaminoxy-2-fluorpropylamin (s. Beispiel 15) wird mit Natriumcyanoborhydrid in analoger Weise wie in Beispiel 10 beschrieben zur 3-Isopropylaminoxy-2-fluorpropylamin reduziert. Das Dihydrochloridsalz wird als gelbliches Pulver erhalten Rf-Wert = 0,59 (Kieselgel/Methylenchlorid:Methanol:Ammoniak 40:10:1). Das Produkt ist mit demjenigen aus Beispiel 11 identisch.

Beispiel 17:

Eine Lösung von 724 mg (4 mMol) 3-Aminoxy-2-fluorpropylamin-dihydrochlorid (s. Beispiel 5) in 10 ml Ethanol und 4 ml 1N Natronlauge wird mit 0,4 ml Benzaldehyd versetzt und 16 Stunden bei Raumtemperatur gerührt. Das Reaktionsgemisch wird eingedampft und der Rückstand zwischen 4 ml 1N Natronlauge und 100 ml Ether verteilt. Die Etherphase wird mit 10 ml gesättigter Kochsalzlösung gewaschen, über Natriumsulfat getrocknet und zur Trockene eingedampft. Der ölige Rückstand wird in 7 ml Essigester aufgenommen und mit 3,7 ml einer 1N Lösung von HCl Gas in Essigester versetzt. Das auskristallisierte 3-Benzyliden aminoxy-2-fluorpropylamin-hydrochlorid wird abgesaugt und getrocknet, Smp. 150-152°, Rf-Wert 0,46 (Kieselgel/Methylenchlorid:Methanol:konz. Ammoniak 300:50:1).

Beispiel 18:

3-Benzylidenaminoxy-2-fluorpropylamin (s. Beispiel 17) wird mit Natriumcyanoborhydrid in analoger Weise wie in Beispiel 10 beschrieben zu 3-Benzylaminoxy-2-fluorpropylamin reduziert, Rf-Wert 0,35 (Kieselgel/Methylenchlorid:Methanol:konz. Ammoniak 40:10:1).

Beispiel 19:

Eine Lösung von 5,43 g (30 mMol) 3-Aminoxy-2-fluorpropylamin-dihydrochlorid (s. Beispiel 5) in 60 ml Ethanol und 30 ml 1N Natronlauge wird mit 3,2 ml Salicylaldehyd versetzt und 16 Stunden bei Raumtemperatur gerührt. Das Reaktionsgemisch wird eingedampft und der Rückstand zwischen 30 ml 1N Natronlauge und 100 ml Ether verteilt. Die Etherphase wird mit 30 ml gesättigter Kochsalzlösung gewaschen, über Natriumsulfat getrocknet und zur Trockene eingedampft. Der ölige Rückstand wird in 70 ml Essigester aufgenommen und mit 14 ml einer 2,2N-Lösung von HCl-Gas in Essigester versetzt. Das auskristallisierte 3-(2-Hydroxybenzyliden-aminoxy)-2-fluorpropylamin-hydrochlorid wird abgesaugt und getrocknet, Smp. 174-175°, Rf-Wert 0,33 (Kieselgel/Methylenchlorid:Methanol:konz. Ammoniak 300:50:1)

Beispiel 20:

Eine Suspension von 2,1 g (10 mMol) 3-(1-Ethoxyethylidenaminoxy)-2-hydroxypropylamin-hydrochlorid (U.S. Patent Nr. 4 404 384) in 20 ml Methylenchlorid wird portionsweise innerhalb von 15 min mit 2,7 g (13 mMol) Phosphorpentachlorid versetzt und während 2 h bei Raumtemperatur gerührt. Nach der Zugabe von 2 ml Wasser wird das Reaktionsgemisch weitere 2 h bei Raumtemperatur nachgerührt und eingedampft. Der Rückstand wird analog Beispiel 1 mit stark basischem Ionenaustauscher MWA-1 (Dow Chemicals) behandelt, wobei man 3-Aminoxy-2-chlorpropylamin-dihydrochlorid erhält, Rf-Wert 0,31 (Kieselgel/Methylenchlorid:Methanol:konz. Ammoniak 40:10:1).

Beispiel 20a:

Analog Beispiel 2 wird N,N′-Di-BOC-3-aminoxy-2-chlorpropylamin mit Chlorwasserstoff in Essigester behandelt, wobei man 3-Aminoxy-2-chlorpropylamin-dihydrochlorid erhält, Rf-Wert 0,31 (Kieselgel/Methylenchlorid:Methanol:konz. Ammoniak 40:10:1).

Das Ausgangsmaterial wird wie folgt hergestellt:

Das Reaktionsgemisch aus Beispiel 20 wird nach der Hydrolyse durch Zugabe von Wasser mit 10 g Natriumhydrogencarbonat vorsichtig versetzt und analog Beispiel 2 mit 2,5 g Di-tert.-butyldicarbonat zum N,N′-Di-BOC-3-aminoxy-2-chlorpropylamin umgesetzt. Die Reinsubstanz wird als farblose Kristalle vom Smp. 111-113° isoliert.

Beispiel 21:

Eine Lösung von 450 mg (2,5 mMol) 3-Aminoxy-2-fluorpropylamin-dihydrochlorid (s. Beispiel 5) in 5 ml Ethanol und 2,5 ml 1N Natronlauge wird mit 0,4 ml 2-Oxopropionsäureethylester versetzt und 16 Stunden bei Raumtemperatur gerührt. Das Reaktionsgemisch wird eingedampft und der Rückstand zwischen Wasser und Ether verteilt. Die untere Phase wird eingeengt und lyophilisiert. Man erhält das 3-(1-Ethoxycarbonylethylidenaminoxy)-2-fluorpropylamin-hydrochlorid als weisses Pulver, das 1 Mol Kochsalz enthält, Rf-Wert 0,30 (Kieselgel/Methylenchlorid:Methanol:konz. Ammoniak 300:50:1).

Beispiel 21a:

450 mg (2,5 mMol) 3-Aminoxy-2-fluorpropylamin-dihydrochlorid wird in analoger Weise wie in Beispiel 21 beschrieben mit 0,5 ml 2-Oxopropionsäuremethylester umgesetzt, wobei man 3-(1-Methoxycarbonylethylidenaminoxy)-2-fluorpropylamin-hydrochlorid als weisses Pulver erhält, Smp. 150° (Zers.).

Beispiel 22:

3-Aminoxy-2-fluorpropylamin (s. Beispiel 5) wird mit 2-Oxopropionsäure in analoger Weise wie in Beispiel 3 beschrieben umgesetzt, wobei man 3-(1-Carboxyethylidenaminoxy)-2-fluorpropylamin erhält.

Beispiel 23:

3-Aminoxy-2-fluorpropylamin (s. Beispiel 5) wird mit Methoxyaceton in analoger Weise wie in Beispiel 21 beschrieben umgesetzt, wobei man 3-(1-Methoxymethylethylidenaminoxy)-2-fluorpropylamin erhält, Smp. 70°, Rf-Wert 0,33 (Kieselgel/Methylenchlorid:Methanol:konz. Ammoniak 300:50:1).

Beispiel 24:

3-Aminoxy-2-fluorpropylamin (s. Beispiel 5) wird mit Acrolein in analoger Weise wie in Beispiel 3 beschrieben umgesetzt, wobei man 3-(Prop-2-en-1-ylidenaminoxy)-2-fluorpropylamin erhält, Rf-Wert 0,35 (Kieselgel/Methylenchlorid:Methanol:konz. Ammoniak 300:50:1).

Beispiel 24a:

3-Aminoxy-2-hydroxypropylamin-dihydrochlorid wird mit Acrolein in analoger Weise wie in Beispiel 21 beschrieben umgesetzt, wobei man 3-(2-Propenylidenaminoxy)-2-hydroxypropylamin-hydrochlorid erhält, Rf-Wert 0,23 (Kieselgel/Methylenchlorid:Methanol:konz. Ammoniak 40:10:1).

Beispiel 25:

3-Aminoxy-2-fluorpropylamin (s. Beispiel 5) wird mit Methylvinylketon in analoger Weise wie in Beispiel 3 beschrieben umgesetzt, wobei man 3-(1-Methylprop-2-en-1-ylidenaminoxy)-2-fluorpropylamin erhält, Rf-Wert 0,35 (Kieselgel/Methylenchlorid:Methanol:konz. Ammoniak 300:50:1).

Beispiel 26:

Eine Lösung von 900 mg (5,0 mMol) 3-Aminoxy-2-fluorpropylamin-dihydrochlorid (s. Beispiel 5) in 10 ml Ethanol und 5,0 ml 1N Natronlauge wird mit 0,5 ml (5,5 mMol) Ethylmethylketon versetzt und 16 Stunden bei Raumtemperatur gerührt. Das Reaktionsgemisch wird eingedampft und der Rückstand zwischen Wasser und Ether verteilt. Die untere Phase wird eingeengt und lyophilisiert. Man erhält das 3-(1-Methyl-1-propylidenaminoxy)-2-fluor-propylamin-hydrochlorid als weisses Pulver, das 1 Mol Kochsalz enthält, Rf-Wert 0,31 (Kieselgel/Methylenchlorid:Methanol:konz. Ammoniak 300:50:1).

Beispiel 26a:

Eine Lösung von 1,8 g (10 mMol) 3-Aminoxy-2-hydroxypropylamin-dihydrochlorid in 20 ml Ethanol wird in analoger Weise wie in Beispiel 21 beschrieben mit 10 ml 1N NaOH und 0,99 ml (11 mMol) Ethyl methylketon umgesetzt. Man erhält das 3-(1-Methyl-1-propylidenaminoxy)-2-hydroxypropylamin-hydrochlorid als weisses Pulver, das 1 Mol Kochsalz enthält, RF-Wert 0,29 (Kieselgel/Methylenchlorid:Methanol:konz. Ammoniak 150:50:1).

Beispiel 27:

Eine Lösung von 850 mg N-BOC-N-Propargyl-3-(1-ethoxyethylidenaminoxy)-2-hydroxypropylamin in 5 ml Dioxan und 5 ml 2N Salzsäure wird 30 min am Rückfluss gekocht und anschliessend eingedampft. Der Rückstand wird in wenig Wasser gelöst, klar filtriert und gefriergetrocknet. Man erhält so das N-Propargyl-3-aminoxy-2-hydroxypropylamindihydrochlorid als braunes amorphes Pulver, Rf-Wert = 0,3 (Kieselgel/Methylenchlorid:Methanol:konz. Ammoniak 150:50:1).

Das Ausgangsmaterial wird wie folgt hergestellt:

Eine Lösung von 2,12 g (13,3 mMol) O-2,3-Epoxypropyl-acethydroxamsäureethylester in 10,5 ml Isopropanol wird mit 8,5 ml (133,3 mMol) Propargylamin versetzt und 3 Tage bei Raumtemperatur stehengelassen. Das Reaktionsgemisch wird eingedampft und anschliessend in 30 ml Methylenchlorid aufgenommen. Man gibt nun unter Eiskühlung tropfenweise eine Lösung von 16,6 g Di-tert.-butyldicarbonat in 30 ml Methylenchlorid zu und lässt 24 h bei Raumtemperatur nachreagieren. Nach der Aufarbeitung in analoger Weise wie im Beispiel 2 beschrieben und Chromatographie an Kieselgel in Methylenchlorid/Essigester (85:15) erhält man das N-BOC-N-propargyl-3-(1-ethoxyethylidenaminoxy)-2-hydroxypropylamin als gelbes Oel, Rf-Wert = 0,38 (Kieselgel/Methylenchlorid:Essigester 85:15).

Beispiel 27a:

Eine Lösung von 1,15 g (5 mMol) N-Methallyl-3-(1-ethoxyethylidenaminoxy)-2-hydroxypropylamin in 9 ml Dioxan und 9 ml 2N Salzsäure wird 30 Minuten am Rückfluss gekocht, filtriert und zur Trockene eingedampf. Der Rückstand wird aus Ethanol/Ether kristallisiert. Man erhält so das N-Methallyl-3-aminoxy-2-hydroxypropylamin-dihydrochlorid als weisses hygroskopisches Pulver, Rf-Wert = 0,33 (Kieselgel/Methylenchlorid:Methanol:konz. Ammoniak 40:10:1).

Das Ausgangsmaterial wird wie folgt hergestellt:

Eine Lösung von 2,82 g (17,5 mMol) 0-2,3-Epoxypropyl-acethydroxamsäureethylester in 20 ml Methanol wird mit 8,8 g 2-Methallylamin-hydrochlorid und 74,4 ml 1,1N Natriummethylatlösung versetzt und 5 Stunden am Rückfluss gekocht. Das Reaktionsgemisch wird filtriert und eingedampft. Der Rückstand wird über 250 g Kieselgel mit Methylenchlorid:Methanol:konz. Ammoniak 150:50:1 chromatographiert, wobei das N-Methallyl-3-(1-ethoxyethylidenaminoxy)-2-hydroxypropylamin erhalten wird, Rf-Wert 0,45 (Kieselgel/Methylenchlorid:Methanol:konz. Ammoniak 300:50:1).

Beispiel 27b:

Eine Lösung von 2,75 g (12,5 mMol) N-Allyl-3-(1-ethoxyethylidenaminoxy)-2-hydroxypropylamin in 30 ml 2N Salzsäure wird 40 Minuten am Rückfluss gekocht, filtriert und zur Trockene eingedampft. Man erhält so das N-Allyl-3-aminoxy-2-hydroxypropylamin-dihydrochlorid als gelbes Oel, Rf-Wert = 0,23 (Kieselgel/Methylenchlorid:Methanol:konz. Ammoniak 40:10:1).

Das Ausgangsmaterial wird wie folgt hergestellt:

Eine Lösung von 2,82 g (17,5 mMol) 0-2,3-Epoxypropyl-acethydroxamsäureethylester in 35 ml Methanol wird mit 6,1 ml (81,8 mMol) Allylamin versetzt und 16 Stunden bei 50° gerührt. Das Reaktionsgemisch wird eingedampft und der Rückstand wird über 250 g Kieselgel mit Methylenchlorid:Methanol:konz. Ammoniak 150:50:1 chromatographiert, wobei das N-Allyl-3-(1-ethoxyethylidenaminoxy)-2-hydroxypropylamin erhalten wird, Rf-Wert 0,60 (Kieselgel/Methylenchlorid:Methanol:konz. Ammoniak 150:50:1).

Beispiel 28:

Eine Lösung von 543 mg (3 mMol) 3-Aminoxy-2-fluorpropylamin-dihydrochlorid in 8 ml Ethanol wird mit 3 ml 1N NaOH und 0,54 g (3 mMol) Glucose versetzt, 16 Stunden bei Raumtemperatur stehen gelassen und zur Trockene eingedampft. Der Rückstand wird in 10 ml Wasser aufgenommen, filtriert und lyophilisiert. Man erhält das 0-(3-Amino-2-fluorpropyl)-glucoseoxim-hydrochlorid, Rf-Wert 0,16 (Kieselgel/n-Butanol:Pyridin:konz. Ammoniak:Wasser 200:120:30:150).

Beispiel 29:

Eine Lösung von 212 mg (1 mMol) 3-(1-Ethoxyethylidenaminoxy)-2S-hydroxypropylamin-hydrochlorid in 3 ml 1N Salzsäure wird 1 h am Rückfluss gekocht und anschliessend eingedampft. Der Rückstand wird aus Ethanol/Ether kristallisiert, wobei man das 3-Aminoxy-2S-hydroxypropylamin-dihydrochlorid erhält, Smp. 150-155°, $[\alpha]_D^{20}$ +3,0° (c=1,009 in Wasser).

Die Ausgangsverbindungen werden wie folgt hergestellt:

Eine Lösung von 2,25 g (22 mMol) Acethydroxamsäureethylester und 5,0 g (22 mMol) 2R-p-Toluolsulfonsäureglycidylester (≙ 3-Tosyloxy-1,2R-epoxypropan) in 50 ml Aceton wird bei 55° tropfenweise mit 4,4 ml 5H Natronlauge versetzt und 1 h am Rückfluss gekocht. Nach der Abkühlung wird filtriert und das Filtrat im Vakuum eingedampft. Der Rückstand wird in 50 ml Ether aufgenommen. Man wäscht diese Lösung zweimal mit 10 ml Wasser, trocknet über Magnesiumsulfat und dampft wieder ein. Der erhaltene ölige O-2S,3-Epoxypropyl-acethydroxamsäureethylester wird im Vakuum destilliert, Kp. 85-90°/18 mbar.

3,2 g (20 mMol) O-2S,3-Epoxypropyl-acethydroxamsäureethylester werden mit 100 ml konz. Ammoniak versetzt, 16 h verschlossen bei Raumtemperatur gerührt und anschliessend zur Trockne eingedampft. Der Rückstand wird noch einmal in 50 ml Wasser aufgenommen und mit 1N Salzsäure auf pH 5,5 gestellt. Die Lösung wird eingedampft und der kristalline Rückstand von 3-(1-Ethoxyethylidenaminoxy)-2S-hydroxypropylamin-hydrochlorid aus Ethanol/Essigester umkristallisiert, Smp. 103-106°.

Beispiel 30:

Analog Beispiel 29 wird ausgehend von Acethydroxamsäureethylester und 2S-p-Toluolsulfonsäureglycidylether (≙ 3-Tosyloxy-1,2S-epoxypropan) das 3-Aminoxy-2R-hydroxypropylamin-dihydrochlorid erhalten, Smp. 157-158°; $[\alpha]_D^{20}$ -3,0° (c=1,039 in Wasser).

Beispiel 31:

Eine Lösung von 1,2 g (4,1 mMol) N-[3-(1-Ethoxy-ethylidenaminoxy)-2-hydroxypropyl]-N-BOC-hydroxylamin in 10 ml Dioxan und 5 ml 2N HCl wird 1 Stunde am Rückfluss gekocht und zur Trockene eingedampft. Man erhält so das N-(3-Aminoxy-2-hydroxypropyl)-hydroxylamin-dihydrochlorid, Rf-Wert 0,05 (Kieselgel/Methylenchlorid:Methanol:konz. Ammoniak 40:10:1).

Das Ausgangsmaterial wird wie folgt hergestellt:

Eine Lösung von 4,8 g (30 mMol) 0-(2,3-Epoxypropyl)-acethydroxamsäureethylester in 40 ml Methanol wird zu einem Gemisch von 10,4 g (0,15 Mol) Hydroxylamin-hydrochlorid und 5,9 g Natronlauge in 40 ml Methanol gegeben und 24 Stunden bei Raumtemperatur gerührt. Das Reaktionsgemisch wird eingedampft, in je 25 ml Wasser und THF aufgenommen und mit 8,0 g Di-tert.-butyl-dicarbonat (Fluka) in 40 ml THF umgesetzt. Nach der Aufarbeitung und chromatographischer Reinigung über 250 g Kieselgel mit Hexan: Essigester 1:1 wird das N-[3-(1-Ethoxy-ethylidenaminoxy)-2-hydroxypropyl]-N-BOC-hydroxylamin als blassgelbes Oel erhalten, Rf-Wert 0,05 (Kieselgel/Hexan:Essigester 1:1).

Beispiel 32:

2,54 g (11,53 mMol) 0-[3-(2-Hydroxyethylamino)-2-hydroxypropyl]-acethydroxamsäure-ethylester in 50 ml 2N Salzsäure werden unter Rühren während 3 Stunden am Rückfluss gekocht. Nach dem Abkühlen wird das Reaktionsgemisch im Wasserstrahlvakuum zur Trockene eingedampft, wobei das 0-[3-(2-Hydroxyethylamino)-2-hydroxypropyl]-hydroxylamin-dihydrochlorid als ein blassgelbes Oel erhalten wird, Rf-Wert 0,43 (Kieselgel/Methylenchlorid:Methanol:konz. Ammoniak 5:3:1).

Das Ausgangsmaterial wird wie folgt hergestellt:

Eine Lösung von 6,4 g (40 mMol) 0-(2,3-Epoxypropyl)-acethydroxamsäureethylester und 8 ml Ethanolamin in 80 ml Isopropanol werden 18 Stunden bei 60° gerührt und zur Trockene eingedampft. Der erhaltene Rückstand wird über 250 g Kieselgel mit dem System Methylenchlorid:Methanol:konz. Ammoniak 300:50:1 chromatographiert. Die Fraktionen mit dem Rf-Wert 0,1 (im gleichen System) werden vereinigt und eingedampft. Das zurückgebliebene gelbe Oel stellt das 0-[3-(2-Hydroxyethylamino)-2-hydroxypropyl]-acethydroxamsäure-ethylester dar.

Beispiel 33:

Ein Gemisch von 6,5 g (34 mMol) 0-(3-Methylamino-2-hydroxypropyl)-acethydroxamsäure-ethylester in 150 ml 2N Salzsäure wird 4 Stunden am Rückfluss gekocht und anschliessend zur Trockene eingedampft. Der wachsartige Rückstand von N-(3-Aminoxy-2-hydroxypropyl)-methylamin-dihydrochlorid wird in wenig Wasser aufgenommen, durch Filtration über 150 ml Dowex 1x4 Ionenaustauscherharz (in basischer Form) in die freie Base umgewandelt und als Oxalatsalz kristallisiert, Smp. 130-133° (aus Methanol), Rf-Wert 0,43 (Kieselgel/Methylenchlorid:Methanol:konz. Ammoniak 40:10:1).

Das Ausgangsmaterial wird wie folgt hergestellt:

Eine Lösung von 8,0 g (50 mMol) 0-(2,3-Epoxypropyl)-acethydroxamsäureethylester und 50 ml einer 33 %igen ethanolischen Methylaminlösung in 100 ml Isopropanol werden 4 Stunden bei 85° gerührt und zur Trockene eingedampft. Der erhaltene Rückstand wird über 250 g Kieselgel mit Essigester chromatographiert. Die Frakfionen mit dem Rf-Wert 0,21 (Kieselgel/Methylenchlorid:Methanol:konz. Ammoniak 150:50:1) werden vereinigt und eingedampft. Das zurückgebliebene gelbe Oel stellt den 0-(3-Methylamino-2-hydroxypropyl)-acethydroxamsäure-ethylester dar.

Beispiel 34:

Ein Gemisch von 0,8 g Bis-1,7-ethoxyethylidenaminoxy-4-aza-2,6-dihydroxyheptan in 20 ml 2N Salzsäure wird 0,5 Stunden am Rückfluss gekocht und anschliessend zur Trockene eingedampft. Der wachsartige hygroskopische Rückstand von Bis-1,7-aminoxy-4-aza-2, 6-dihydroxy-heptantrihydrochlorid wird aus Methanol/Ether kristallisiert, Rf-Wert 0,44 (Kieselgel/Methylenchlorid:Methanol:konz. Ammoniak 5:3:1).

Das Ausgangsmaterial wird wie folgt hergestellt:

Eine Lösung von 6,4 g (40 mMol) 0-(2,3-Epoxypropyl)-acethydroxamsäureethylester und 7,04 g 0-(3-Amino-2-hydroxypropyl)-acethydroxamsäureethylester in 100 ml Isopropanol werden 3,5 Stunden bei 70°

gerührt und zur Trockene eingedampft. Der erhaltene Rückstand wird über 700 g Kieselgel mit Essigester chromatographiert. Die Fraktionen mit dem Rf-Wert 0,30 (Kieselgel/Essigester) werden vereinigt und eingedampft. Das zurückgebliebene gelbe· Oel stellt das Bis-1,7-ethoxyethylidenaminoxy-4-aza-2,6-dihydroxy-heptan dar.

Beispiel 35:

Eine Lösung von 0,54 g (3 mMol) 3-Aminoxy-2-fluorpropylamin-dihydrochlorid (s. Beispiel 5) in 8 ml Ethanol und 3 ml 1N Natronlauge wird mit 0,26 ml Cyclopentanon versetzt und 16 Stunden bei Raumtemperatur gerührt. Das Reaktionsgemisch wird eingedampft und der Rückstand zwischen 3 ml 1N Natronlauge und 10 ml Ether verteilt. Die Etherphase wird mit gesättigter Kochsalzlösung gewaschen, über Natriumsulfat getrocknet und zur Trockene eingedampft. Der ölige Rückstand wird in 10 ml Essigester aufgenommen und mit 2,5 ml einer 1N Lösung von HCl Gas in Essigester versetzt. Das auskristallisierte 3-(Cyclopentylidenaminoxy)-2-fluorpropylamin-hydrochlorid wird abgesaugt und getrocknet, Smp. 109-112°.

Beispiel 36:

Eine Lösung von 2,43 g N-BOC-3-Pyridoxalaminoxy-2-fluorpropylamin in 10 ml Methanol wird mit 5 ml 6N methanolischer HCl versetzt und unter Feuchtigkeitsausschluss stehen gelassen. Das auskristallisierte 3-Pyridoxalaminoxy-2-fluorpropylamin-dihydrochlorid wird abgesaugt, mit Essigester gewaschen und getrocknet, Smp. 210-213°.

Das Ausgangsmaterial wird wie folgt hergestellt:

Eine Lösung von 724 mg (4 mMol) 3-Aminoxy-2-fluorpropylamin-dihydrochlorid (s. Beispiel 5) in 13 ml Ethanol wird mit 814 mg (4 mMol) Pyridoxalhydrochlorid und 8 ml 1N Natronlauge versetzt und 16 Stunden bei Raumtemperatur gerührt. Zum Reaktionsgemisch werden weitere 4 ml 1N Natronlauge und eine Lösung von 1,08 g Di-tert.-butyl-dicarbonat in 20 ml THF gegeben. Das Reaktionsgemisch liefert nach konventioneller Aufarbeitung und chromatographischer Reinigung das N-BOC-3-Pyridoxalaminoxy-2-fluor-propylamin als gelben Schaum, Rf-Wert 0,25 (Kieselgel/Methylenchlorid:Methanol 10:1).

Beispiel 37:

Eine Lösung von 1,8 g (10 mMol) 3-Aminoxy-2-hydroxypropylamin-dihydrochlorid in 20 ml Ethanol wird mit 10 ml 1N NaOH und 1,02 ml (11 mMol) Methoxyaceton umgesetzt. Das Reaktionsgemisch wird eingedampft und der Rückstand zwischen Wasser und Ether verteilt. Die untere Phase wird eingeengt und lyophilisiert. Man erhält das 3-(1-Methyl-2-methoxyethylidenaminoxy)-2-hydroxypropylamin-hydrochlorid als weisses Pulver, das 1 Mol Kochsalz enthält, RE-Wert 0,2 (Kieselgel/Methylenchlorid:Methanol:konz. Ammoniak 150:50:1).

Beispiel 38:

Eine Lösung von 1,8 g (10 mMol) 3-Aminoxy-2-hydroxypropylamin-dihydrochlorid in 20 ml Ethanol wird mit 10 ml 1H NaOH und 0,72 ml (10 mMol) Brenztraubensäuremethylester umgesetzt. Das Reaktionsgemisch wird eingedampft und der Rückstand zwischen Wasser und Ether verteilt. Die untere Phase wird eingeengt und lyophilisiert. Man erhält das 3-(1-Methoxycarbonyl-ethylidenaminoxy)-2-hydroxypropylamin-hydrochlorid als weisses Pulver, das 1 Mol Kochsalz enthält; Smp. 136-140°, Rf-Wert 0,38 (Kieselgel/Methylenchlorid:Methanol:konz. Ammoniak 40:10:1).

Beispiel 39:

Eine Lösung von 0,72 g (4 mMol) 3-Aminoxy-2-hydroxypropylamin-dihydrochlorid in 10 ml Ethanol wird mit 4 ml 1H NaOH und 0,72 g 4 mMol) Glucose versetzt, 16 Stunden bei Raumtemperatur stehen gelassen und zur Trockene eingedampft. Der Rückstand wird in 10 ml Wasser aufgenommen, filtriert und lyophilisiert. Man erhält das 0-(3-Amino-2-hydroxypropyl)-glucoseoxim-hydrochlorid, RE-Wert 0,05 (Kieselgel/n-Butanol:Pyridin:konz. Ammoniak:Wasser 200:120:30:150).

Beispiel 40:

Eine Lösung von 750 mg 3-(2-Butylaminoxy)-2-hydroxy-N-BOC-propylamin in 10 ml Ethanol wird mit 5 ml 5,5N HCl/EtOH versetzt, 16 Stunden bei Raumtemperatur stehen gelassen und zur Trockene eingedampft. Man erhält so das 3-(2-Butylaminoxy)-2-hydroxy-propylamindihydrochlorid als hellgelbes viskoses Oel, RE-Wert 0,14 (Kieselgel/Methylenchlorid:Methanol:konz. Ammoniak 40:10:1).

Das Ausgangsmaterial wird wie folgt hergestellt:

Eine Lösung von 1,05 g (3,9 mMol) 3-(1-Methylpropylidenaminoxy)-2-hydroxy-N-BOC-propylamin in 25 ml THF wird mit 2,5 ml Eisessig und 0,27 g (3,9 mMol) Natriumcyanoborohydrid versetzt und 48 Stunden bei Raumtemperatur gerührt. Nach der Zugabe von 4 g Natriumhydrogencarbonat wird das Reaktionsgemisch zur Trockene eingedampf und der Rückstand zwischen Wasser unf Ether verteilt. Die organische Phase wird über Magnesiumsulfat getrocknet, eingedampf und über 75 g Kieselgel mit Hexan:Essigester 1:1 chromatographiert. Man erhält das 3-(2-Butylaminoxy)-2-hydroxy-N-BOC-propylamin als blassgelbes Oel, Rf-Wert 0,10 (Kieselgel/Hexan/Essigester 1:1).

Beispiel 41:

Eine Lösung von 0,72 g (4 mMol) 3-Aminoxy-2-hydroxypropylamin-dihydrochlorid in 10 ml Ethanol wird mit 4 ml 1N NaOH und 0,53 g (4 mMol) Indanon versetzt, 16 Stunden bei 50° gerührt und zur Trockene eingedampf. Der Rückstand wird in 10 ml Wasser gelöst und nach Zugabe von 4 ml 1N NaOH mit Ether extrahiert. Die Etherphase wird mit gesättigter Kocksalzlösung gewaschen, über Natriumsulfat getrocknet und eingedampft. Das so erhaltene 0-(3-Amino-2-hydroxy-propyl)-indanoxim wird aus Ether/Petrolether kristallisiert, Smp. 88-92°, Rf-Wert 0,10 (Kieselgel/Methylenchlorid:Methanol:konz. Ammoniak 300:50:1).

Beispiel 42:

Eine Lösung von 543 mg (3 mMol) 3-Aminoxy-2-fluoropropylamin-dihydrochloride in 8 ml Ethanol wird mit 3 ml 1N NaOH und 471 mg (3 mMol) 4-Cyanoindanon versetxt, 16 Stunden bei 50° gerührt und zur Trockene eingedampf. Der Rückstand wird in 10 ml Wasser gelöst und nach Zugabe von 3ml 1N NaOH mit Ether extrahiert. Die Etherphase wird mit gesättiger Kochsalzlösung gewaschen, über Natrimsulfat getrocknet und eingedampft. Das so erhaltene 0-(3-Amino-2-fluoropropyl)-4-cyanoindanonoxim wird aus Ether/Petrolether kristallisiert, Smp. 90-92°, Rf-Wert 0,46 (Kieselgel/Methylenchlorid:Methanol:konz. Ammoniak 300:50:1).

Beispiel 43:

Eine Lösung von 720 mg (4 mMol) 3-Aminoxy-2-hydroxypropylamin-dihydrochlorid in 10 ml Ethanol wird mit 4 ml 1N NaOH und 0,22 ml (4,4 mMol) Hydroxyaceton umgesetzt. Das Reaktionsgemisch wird eingedampft und der Rückstand zwischen Wasser und Ether verteilt. Die untere Phase wird eingeengt und lyophilisiert. Man erhält 3-(1-Hydroxymethyl ethylidenaminoxy)-2-hydroxypropyl-aminhydrochlorid als braunes Oel, das 1 Mol Kochsalz enthält, Rf-Wert 0,10 (Kieselgel/Methylenchlorid:Methanol:konz. Ammoniak 40:10:1).

Beispiel 44:

Eine Lösung von 1,22 g (3,2 mMol) 3-(1-Ethoxy-ethylidenaminoxy)-2-hydroxy-N-BOC-N-benzyloxy-propylamin in 30 ml 1N HCl wird 1 Stunde am Rückfluss gekocht und zur Trockene eingedampft. Der Rückstand wird aus Essigester kristallisiert, mit Essigester/Ether gewaschen und getrocknet. Man erhält so das 3-Aminoxy-2-hydroxy-N-benzyloxy-propylamin-dihydrochlorid, Smp. 140-143°.

Das Ausgangsmaterial wird wie folgt hergestellt:

Eine Lösung von 1,6 g (10 mMol) 0-(2,3-Epoxypropyl)-acethydroxamsäureethylester in 16 ml Methanol

wird zu einer Lösung von 16 g (0,1 Mol) O-Benzylhydroxylamin-hydrochlorid in 70 ml Methanol und 90 ml 1,2N Natriummethylat getropft und 24 Stunden bei Raumtemperatur gerührt. Das Reaktionsgemisch wird eingedampft in 100 ml THF aufgenommen und mit 28,8 g Di-tert.-butyl-dicarbonat (Fluka) in 100 ml THF umgesetzt. Nach konventioneller Aufarbeitung und chromatographischer Reinigung über 700 g Kieselgel mit Hexan:Essigester 2:1 wird das 3-(1-Ethoxy-ethylidenaminoxy)-2-hydroxy-N-BOC-N-benzyloxy-propylamin als blassgelbes Oel erhalten, Rf-Wert 0,41 (Kieselgel/Hexan:Essigester 2:1).

Beispiel 45:

Eine Lösung von 362 mg (2 mMol) 3-Aminoxy-2-fluorpropylamin-dihydrochlorid (s. Beispiel 5) in 20 ml 0,1N NaOH wird mit 530 mg (2 mMol) Pyridoxalphosphat versetzt, 30 Minuten bei Raumtemperatur gerührt, filtriert und lyophilisiert. Das erhaltene hygroskopische Lyophilisat stellt das 3-Amino-2-fluor-propyloxyimino-pyridoxalphosphathydrochlorid dar, das 1 Mol Kochsalz enthält, Rf-Wert 0,13 (Kieselgel/Methylethylketon:Ethanol:Wasser:konz. Ammoniak 15:5:5:5; Pyridoxalphosphat Rf-Wert 0,26).

Beispiel 46:

Eine Lösung von 180 mg (1 mMol) 3-Aminoxy-2-hydroxypropylamin-dihydrochlorid in 10 ml 0,1N NaOH wird mit 265 mg (1 mMol) Pyridoxalphosphat versetzt, 30 Minuten bei Raumtemperatur gerührt, filtriert und lyophilisiert. Das erhaltene hygroskopische Lyophilisat stellt das 3-Amino-2-hydroxy-propyloxyimino-pyridoxalphosphat-hydrochlorid dar, das 1 Mol Kochsalz enthält, Rf-Wert 0,10 (Kieselgel/Methylethylketon:Ethanol:Wasser:konz. Ammoniak 15:5:5:5; Pyridoxalphosphat Rf-Wert 0,26).

Beispiel 47:

Eine Lösung von 4,21 g (13,7 mMol) 3-(1-Ethoxy-ethylidenaminoxy)-2-hydroxy-N-BOC-N-methoxy-propylamin in 130 ml 1N HCl wird 1 Stunde am Rückfluss gekocht und zur Trockene eingedampft. Der Rückstand wird aus Ethanol kristallisiert, mit Essigester/Ether gewaschen und getrocknet. Man erhält so das 3-Aminoxy-2-hydroxy-N-methoxy-propylamindihydrochlorid, Smp. 142-152°.

Das Ausgangsmaterial wird wie folgt hergestellt:

Eine Lösung von 3,2 g (20 mMol) 0-(2,3-Epoxypropyl)-acethydroxamsäureethylester in 30 ml Methanol wird zu einer Lösung von 25 g (0,3 Mol) O-Methylhydroxylamin-hydrochlorid in 100 ml Methanol und 300 ml 1N Natriummethylat getropft und 24 Stunden bei Raumtemperatur gerührt. Das Reaktionsgemisch wird eingedampft, in je 150 ml Wasser und THF aufgenommen und mit 50 g Di-tert.-butyl-dicarbonat in 150 ml THF umgesetzt. Nach der Aufarbeitung und chromatographischer Reinigung über 750 g Kieselgel mit Hexan:Essigester 3:1 wird das 3-(1-Ethoxy-ethylidenaminoxy)-2-hydroxy-N-BOC-N-methoxy-propylamin als blassgelbes Oel erhalten, Rf-Wert 0,2 (Kieselgel/Hexan:Essigester 3:1).

Beispiel 48:

Eine Lösung von 5,0 g (24,7 mMol) 3-(1-Ethoxy-ethylidenaminoxy)-1-azidoisopropanol in 50 ml 2H HCl wird 1 Stunde am Rückfluss gekocht und zur Trockene eingedampft. Man erhält so das 3-Aminoxy-1-azido-isopropanol-hydrochlorid als gelb-braunes Oel, Rf-Wert 0,35 (Kieselgel/Methylenchlorid:Methanol 10:1), IR (Methylenchlorid): 2100 $cm^{-1}$.

Das Ausgangsmaterial wird wie folgt hergestellt:

Eine Lösung von 4,0 g (25 mMol) 0-(2,3-Epoxypropyl)-acethydroxamsäureethylester in 50 ml Ethylenglycolmonomethylether und 2,5 ml Wasser wird mit 3,25 g (50 mMol) Hatriumazid und 1,35 g (25 mMol) Ammoniumchlorid versetzt und 12 Stunden bei 130° gerührt. Nach dem Abkühlen wird das Reaktionsgemisch mit 500 ml Essigester verdünnt, filtriert und eingedampft. Der Rückstand wird in 300 ml Ether aufgenommen, mit Wasser gewaschen, über Magnesiumsulfat getrocknet und vom Lösungsmittel befreit. Man erhält so das 3-(1-Ethoxyethylidenaminoxy)-1-azido-isopropanol als gelbes Oel, Rf-Wert 0,05 (Kieselgel/Hexan:Essigester 1:1); IR (Methylenchlorid): 2100, 3200-3650 $cm^{-1}$.

Beispiel 49:

Eine Lösung von 0,84 g (5 mMol) 3-Aminoxy-1-azido-isopropanol-hydrochlorid in 5 ml Ethanol und 5 ml 1N alkoholischer HCl wird in Gegenwart von 0,1 g 5 %iger Pd/C mit Wasserstoff hydriert. Nach beendigter Reduktion und Abtrennung des Katalysators wird das Filtrat eingedampft und der Rückstand aus Alkohol/Ether kristallisiert. Man erhält so das 3-Aminoxy-2-hydroxypropylamin-dihydrochlorid, Smp. 153-156°

Beispiel 50:

200 mg (1,1 mMol) 3-Aminoxy-2-fluorpropylamin-dihydrochlorid in 1,1 ml 1H NaOH und 4 ml Ethanol werden mit 180 mg (1,15 mMol) Chinolin-4-aldehyd versetzt und 22 Stunden bei 50° gerührt. Die Lösung wird eingeengt und der Rückstand an Opti-Up C-12 mit Wasser chromatographiert. Die das Produkt enthaltenden Fraktionen werden vereinigt, zur Trockene eingeengt und der Rückstand wird mit Ether verrieben. Man lässt über Nacht im Tiefkühlschrank stehen und nutscht den Niederschlag ab. Man erhält 4-(3-Amino-2-fluoro-propyloxyiminomethyl)-chinolin als farbloses Pulver.
FAB-MS: $(M+H)^+$ = 248.
IR(KBr) u.a.: 1625, 1598, 1570 $cm^{-1}$.

Beispiel 51:

114,7 mg (0,64 mMol) 3-Aminoxy-2-hydroxypropylamin-dihydrochlorid in 1 ml Ethanol werden mit 0,64 ml 1H NaOH versetzt gefolgt von 100 mg Difluormethyl-phenylketon (vgl. EP 298 478). Nach 20-stündigem Rühren bei 50° gibt man weitere 28,7 mg 3-Aminoxy-2-hydroxypropylamindihydrochlorid hinzu, gefolgt von 0,16 ml 1N NaOH und 1 ml Ethanol. Man erhitzt weitere 2,5 Stunden bei 80°, engt das Reaktionsgemisch am Rotavap ein und chromatographiert den Rückstand an Opti-Up C-12-Kieselgel (Laufmittel: Wasser). Die das Produkt 3-(2,2-Difluor-1-phenyl-ethylidenaminoxy)-2-hydroxy-propylamin-hydrochlorid enthaltenden Fraktionen werden eingeengt und am Hochvakuum getrocknet.
FAB-MS: $(M+H)^+$ = 245.
IR (KBr) u.a.: 1604, 1526, 1488, 1457 $cm^{-1}$.

Beispiel 52:

Auf analoge Weise wie in Beispiel 51 beschrieben erhält man ausgehend von 3-Aminoxy-2-fluorpropylamin-dihydrochlorid und Difluormethylphenylketon 3-(2,2-Difluor-1-phenyl-ethylidenaminoxy)-2-fluorpropylaminhydrochlorid.
FAB-MS: $(M+H)^+$ = 247.
IR (Nujol) u.a.: 1600, 1496, 1446, 1369 $cm^{-1}$.

Beispiel 53:

3-Aminoxy-2-fluorpropylamin-dihydrochlorid kann auch folgendermassen hergestellt werden:

Ein Gemisch von 0,25 g (0,68 mMol) N-[2-Fluor-3-phthalimidopropyloxy]-phthalimid, 2 ml Wasser und 2 ml konz. Salzsäure wird 2 Stunden unter Rückfluss erhitzt. Man kühlt auf 0° ab, filtriert, wäscht das Nutschgut mit wenig Wasser und dampft das Filtrat im Vakuum ein. Der kristalline Rückstand wird zweimal mit je 10 ml Ethanol zur Trockene eingedampft und anschliessend in 10 ml Ethanol suspendiert. Man filtriert, wäscht das Kristallisat mit Ether und trocknet es bei 70° im Hochvakuum. Die erhaltene Titelverbindung schmilzt bei 206-207°.

Die Ausgangsverbindung wird wie folgt hergestellt:

a) N-(3-Hydroxy-2-fluorpropyl)-phthalimid

Ein Gemisch von 2,5 g (0,0151 Mol) 3-Brom-2-fluor-propan-1-ol (95 %) [Foster et al., J. Med. Chem. 24,

1399 (1981) bzw. I. Chehidi et al., Tetrahedron Letters 30, 3167 (1989)] und 3,243 g (0,0175 Mol) Phthalimid-Kalium in 10 ml Dimethylformamid wird 6 Stunden bei 100° gerührt. Man kühlt auf Raumtemperatur ab, filtriert, dampft das Filtrat im Vakuum ein und verteilt den ölförmigen Rückstand zwischen Wasser und Methylenchlorid. Die über Natriumsulfat getrocknete organische Phase wird eingedampft und der Rückstand aus Essigester-Hexan kristallisiert. Die erhaltene Titelverbindung schmilzt bei 97-99°.

b) N-(2-Fluor-3-phthalimidopropyloxy]-phthalimid

Zu einer Lösung von 0,335 g (0,0015 Mol) N-(3-Hydroxy-2-fluorpropyl)-phthalimid, 0,245 g (0,0015 Mol) H-Hydroxy-phthalimid und 0,394 g (0,0015 Mol) Triphenylphosphin in 5 ml Tetrahydrofuran gibt man unter Rühren 0,273 g (0,00157 Mol) Azodicarbonsäure-diethylester. Man rührt 2 Stunden bei Raumtemperatur, verdünnt die erhaltene Suspension mit ca. 5 ml Tetrahydrofuran, kühlt auf 0° ab, filtriert und wäscht das Kristallisat mit Tetrahydrofuran. Nach Trocknen im Hochvakuum schmilzt die erhaltene Titelverbindung bei 184-185°.

Beispiel 54:

Kapseln enthaltend 0,25 g Wirkstoff, z.B. eine der Verbindungen der Beispiele 1-53, können wie folgt hergestellt werden:

| Zusammensetzung (für 5000 Kapseln) | |
|---|---|
| Wirkstoff | 1250 g |
| Talk | 180 g |
| Weizenstärke | 120 g |
| Magnesiumstearat | 80 g |
| Laktose | 20 g |

Die pulverförmigen Substanzen werden durch ein Sieb mit einer Maschenweite von 0,6 mm getrieben und gemischt. Portionen von je 0,33 g des Gemisches werden mittels einer Kapselfüllmaschine in Gelatine-Kapseln abgefüllt.

**Ansprüche**

1. Verwendung von Verbindungen der Formel I

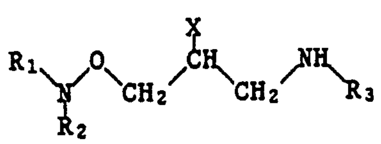

(I)

worin X Halogen, Hydroxy, Niederalkoxy, Acyloxy, Niederalkylsulfonyloxy oder Arylsulfonyloxy bedeutet; $R_1$ und $R_2$ unabhängig voneinander Wasserstoff, Niederalkyl, Cycloalkyl, Arylniederalkyl oder Hetarylniederalkyl bedeuten, oder $R_1$ und $R_2$ zusammen für einen Rest

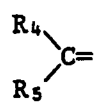

stehen, worin $R_4$ und $R_5$ unabhängig voneinander Wasserstoff, Niederalkyl, Hydroxyniederalkyl, Halogenniederalkyl, Niederalkoxyniederalkyl, Niederalkenyl, Niederalkinyl, Cycloalkyl, Arylniederalkyl, Hetarylniederalkyl, Aryl, Hetaryl, Carboxy oder Niederalkoxycarbonyl bedeuten, oder worin $R_4$ Polyhydroxyniederalkyl bedeutet und $R_5$ für Wasserstoff oder Hydroxymethyl steht, oder worin $R_4$ und $R_5$ zusammen $C_2$-$C_7$-Alkylen oder gegebenenfalls substituiertes Benzo-$C_4$-$C_6$-alkylen bedeuten; und $R_3$ für Wasserstoff, gegebenenfalls durch Hydroxy oder Halogen und/oder Aminoxy substituiertes Niederalkyl, Niederalkenyl, Niederalkinyl oder gegebenenfalls verethertes Hydroxy steht; und pharmazeutisch annehmbaren Salzen davon, zur Herstellung eines Arzneimittels für die therapeutische Behandlung von Krankheiten des menschlichen oder tierischen Körpers, die auf eine Hemmung der Ornithindecarboxylase ansprechen.

2. Verwendung gemäss Anspruch 1 von Verbindungen der Formel I, worin X Halogen, Hydroxy, Niederalkoxy, Niederalkylsulfonyloxy oder Phenylsulfonyloxy bedeutet; $R_1$ und $R_2$ unabhängig voneinander Wasserstoff, Niederalkyl, $C_3$-$C_8$-Cycloalkyl oder Phenylniederalkyl bedeuten, oder $R_1$ und $R_2$ zusammen für einen Rest

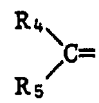

stehen, worin $R_4$ und $R_5$ unabhängig voneinander Wasserstoff, Niederalkyl, Halogenniederalkyl, $C_3$-$C_8$-Cycloalkyl, Phenylniederalkyl oder Phenyl bedeuten, oder worin $R_4$ 3-Hydroxy-5-(hydroxymethyl oder phosphonooxymethyl)-2-methyl-4-pyridyl bedeutet und $R_5$ für Wasserstoff steht, oder worin $R_4$ 1,2,3,4-Tetrahydroxybutyl oder 1,2,3,4,5-Pentahydroxypentyl bedeutet und $R_5$ für Wasserstoff oder Hydroxymethyl steht, oder worin $R_4$ Chinolinyl, Carboxy oder Niederalkoxycarbonyl bedeutet und $R_5$ für Wasserstoff oder Niederalkyl steht, oder worin $R_4$ und $R_5$ zusammen $C_2$-$C_7$-Alkylen oder gegebenenfalls durch Cyano substituiertes Benzo-$C_4$-$C_5$-alkylen bedeuten; und $R_3$ für Wasserstoff, Niederalkyl, Niederalkenyl, Niederalkinyl, Niederalkoxy, Benzyloxy oder Hydroxy steht; wobei Phenylgruppen unsubstituiert oder durch Niederalkyl, Halogenniederalkyl, Halogen, Hydroxy, Niederalkoxy, Niederalkanoyloxy und/oder Nitro substituiert sind; und ihren pharmazeutisch annehmbaren Salzen.

3. Verwendung gemäss Anspruch 1 von Verbindungen der Formel I, worin X Fluor, Chlor, Hydroxy, Niederalkylsulfonyloxy oder Phenylsulfonyloxy bedeutet; $R_1$ und $R_2$ unabhängig voneinander Wasserstoff, Niederalkyl, $C_3$-$C_7$-Cycloalkyl oder Phenylniederalkyl bedeuten, oder $R_1$ und $R_2$ zusammen für einen Rest

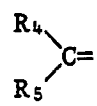

stehen, worin $R_4$ und $R_5$ unabhängig voneinander Wasserstoff, Niederalkyl, Halogenniederalkyl, $C_3$-$C_7$-Cycloalkyl, Phenylniederalkyl oder Phenyl bedeuten, oder worin $R_4$ Chinolinyl, 3-Hydroxy-5-(hydroxymethyl oder phosphonooxymethyl)-2-methyl-4-pyridyl bedeutet und $R_5$ für Wasserstoff steht, oder worin $R_4$ Carboxy oder Niederalkoxycarbonyl bedeutet und $R_5$ für Niederalkyl steht, oder worin $R_4$ und $R_5$ zusammen $-(CH_2)_4-$ oder $-(CH_2)_5-$ bedeuten; und $R_3$ für Wasserstoff, Niederalkyl, Niederalkenyl, Niederalkinyl oder Hydroxy steht; wobei Phenylgruppen unsubstituiert oder durch Niederalkyl, Trifluormethyl, Halogen, Hydroxy, Niederalkoxy und/oder Nitro substituiert sind; und ihren pharmazeutisch annehmbaren Salzen.

4. Verwendung gemäss Anspruch 1 von Verbindungen der Formel I, worin X Fluor, Chlor, Hydroxy oder Methylsulfonyloxy bedeutet, $R_1$ für Wasserstoff steht, $R_2$ Wasserstoff, $C_1$-$C_4$-Alkyl, Benzyl oder 2-Hydroxybenzyl bedeutet, oder $R_1$ und $R_2$ zusammen $C_5$-$C_6$-Cycloalkyliden, $C_1$-$C_4$-Alkyliden, Benzyliden, 2-Hydroxybenzyliden oder 3-Hydroxy-5-(hydroxymethyl oder phosphonooxymethyl)-2-methyl-4-pyridylmethyliden bedeuten, und $R_3$ für Wasserstoff, $C_1$-$C_4$-Alkyl, $C_2$-$C_4$-Alkinyl oder Hydroxy steht; und ihren pharmazeutisch annehmbaren Salzen.

5. Verwendung gemäss Anspruch 1 von 3-Aminoxy-2-hydroxypropylamin und pharmazeutisch annehmbaren Salzen davon.

6. Verbindungen der Formel I

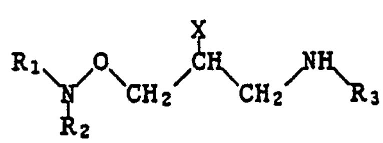

(I),

worin X Halogen, Hydroxy, Niederalkoxy, Acyloxy, Niederalkylsulfonyloxy oder Arylsulfonyloxy bedeutet; $R_1$ und $R_2$ unabhängig voneinander Wasserstoff, Niederalkyl, Cycloalkyl, Arylniederalkyl oder Hetarylniederalkyl bedeuten, oder $R_1$ und $R_2$ zusammen für einen Rest

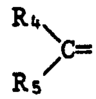

stehen, worin $R_4$ und $R_5$ unabhängig voneinander Wasserstoff, Niederalkyl, Hydroxyniederalkyl, Halogenniederalkyl, Niederalkoxyniederalkyl, Niederalkenyl, Niederalkinyl, Cycloalkyl, Arylniederalkyl, Hetarylniederalkyl, Aryl, Hetaryl, Carboxy oder Niederalkoxycarbonyl bedeuten, oder worin $R_4$ Polyhydroxyniederalkyl bedeutet und $R_5$ für Wasserstoff oder Hydroxymethyl steht, oder worin $R_4$ und $R_5$ zusammen $C_2$-$C_7$-Alkylen oder gegebenenfalls substituiertes Benzo-$C_4$-$C_6$-alkylen bedeuten; und $R_3$ für Wasserstoff, gegebenenfalls durch Hydroxy oder Halogen und/oder Aminoxy substituiertes Niederalkyl, Niederalkenyl, Niederalkinyl oder gegebenenfalls verethertes Hydroxy steht;
mit der Massgabe, dass, wenn X Hydroxy bedeutet und $R_1$ und $R_2$ zusammen für einen Rest

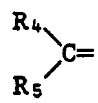

stehen, $R_3$ Wasserstoff bedeutet;
mit der Massgabe, dass, wenn X für Hydroxy steht und $R_3$ Wasserstoff bedeutet, die Reste $R_1$ und $R_2$ zusammen nicht 1,1-Dimethylmethyliden, 1-n-Butyliden oder Nitro-(furanyl oder imidazolyl)-methyliden bedeuten können;
mit der Massgabe, dass, wenn X für Hydroxy steht und $R_3$ Wasserstoff, Hydroxy oder Niederalkyl bedeutet, nicht beide Reste $R_1$ und $R_2$ Wasserstoff bedeuten können;
und mit der Massgabe, dass, wenn X Hydroxy bedeutet und $R_1$ und $R_2$ unabhängig voneinander Wasserstoff, Niederalkyl, Cycloalkyl, Arylniederalkyl oder Hetarylniederalkyl, nicht jedoch beide Wasserstoff, bedeuten, $R_3$ für Wasserstoff, Methyl, Ethyl oder n-Propyl steht; und Salze davon.

7. Verbindungen der Formel I gemäss Anspruch 6, worin X Halogen, Hydroxy, Niederalkoxy, Niederalkylsulfonyloxy oder Phenylsulfonyloxy bedeutet; $R_1$ und $R_2$ unabhängig voneinander Wasserstoff, Niederalkyl, $C_3$-$C_8$-Cycloalkyl oder Phenylniederalkyl bedeuten, oder $R_1$ und $R_2$ zusammen für einen Rest

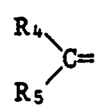

stehen, worin $R_4$ und $R_5$ unabhängig voneinander Wasserstoff, Niederalkyl, Halogenniederalkyl, $C_3$-$C_8$-Cycloalkyl, Phenylniederalkyl oder Phenyl bedeuten, oder worin $R_4$ 3-Hydroxy-5-(hydroxymethyl oder phosphonooxymethyl)-2-methyl-4-pyridyl bedeutet und $R_5$ für Wasserstoff steht, oder worin $R_4$ 1,2,3,4-Tetrahydroxybutyl oder 1,2,3,4,5-Pentahydroxypentyl bedeutet und $R_5$ für Wasserstoff oder Hydroxymethyl steht, oder worin $R_4$ Chinolinyl, Carboxy oder Niederalkoxycarbonyl bedeutet und $R_5$ für Wasserstoff oder Niederalkyl steht, oder worin $R_4$ und $R_5$ zusammen $C_2$-$C_7$-Alkylen oder gegebenenfalls durch Cyano substituiertes Benzo-$C_4$-$C_6$-alkylen bedeuten; und $R_3$ für Wasserstoff, Niederalkyl, Niederalkenyl, Niederalkinyl, Niederalkoxy, Benzyloxy oder Hydroxy steht;
mit der Massgabe, dass, wenn X Hydroxy bedeutet und $R_1$ und $R_2$ zusammen für einen Rest

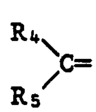

stehen, $R_3$ Wasserstoff bedeutet;
mit der Massgabe, dass, wenn X für Hydroxy steht und $R_3$ Wasserstoff bedeutet, die Reste $R_1$ und $R_2$ zusammen nicht 1,1-Dimethylmethyliden oder 1-n-Butyliden bedeuten können;
mit der Massgabe, dass, wenn X für Hydroxy steht und $R_3$ Wasserstoff, Hydroxy oder Niederalkyl bedeutet,

nicht beide Reste $R_1$ und $R_2$ Wasserstoff bedeuten können;
und mit der Massgabe, dass, wenn X Hydroxy bedeutet und $R_1$ und $R_2$ unabhängig voneinander Wasserstoff, Niederalkyl, Cycloalkyl oder Phenylniederalkyl, nicht jedoch beide Wasserstoff, bedeuten, $R_3$ für Wasserstoff, Methyl, Ethyl oder n-Propyl steht;
wobei Phenylgruppen unsubstituiert oder durch Niederalkyl, Halogenniederalkyl, Halogen, Hydroxy, Niederalkoxy, Niederalkanoyloxy und/oder Nitro substituiert sind; und Salze davon.

8. Verbindungen der Formel I gemäss Anspruch 6, worin X Fluor, Chlor, Hydroxy, Niederalkylsulfonyloxy oder Phenylsulfonyloxy bedeutet; $R_1$ und $R_2$ unabhängig voneinander Wasserstoff, Niederalkyl, $C_3$-$C_7$-Cycloalkyl oder Phenylniederalkyl bedeuten, mit der Massgabe, dass nicht beide Reste $R_1$ und $R_2$ Wasserstoff bedeuten können, wenn X für Hydroxy steht,
oder worin $R_1$ und $R_2$ zusammen für einen Rest

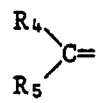

stehen, worin $R_4$ und $R_5$ unabhängig voneinander Wasserstoff, Niederalkyl, Halogenniederalkyl, $C_3$-$C_7$-Cycloalkyl, Phenylniederalkyl oder Phenyl bedeuten, oder worin $R_4$ Chinolinyl, 3-Hydroxy-5-(hydroxymethyl oder phosphonooxymethyl)-2-methyl-4-pyridyl bedeutet und $R_5$ für Wasserstoff steht, oder worin $R_4$ Carboxy oder Niederalkoxycarbonyl bedeutet und $R_5$ für Niederalkyl steht, oder worin $R_4$ und $R_5$ zusammen $-(CH_2)_4-$ oder $-(CH_2)_5-$ bedeuten; und $R_3$ für Wasserstoff steht;
mit der Massgabe, dass, wenn X für Hydroxy steht, die Reste $R_1$ und $R_2$ zusammen nicht 1,1-Dimethylmethyliden oder 1-n-Butyliden bedeuten können;
wobei Phenylgruppen unsubstituiert oder durch Niederalkyl, Trifluormethyl, Halogen, Hydroxy, Niederalkoxy oder Niederalkanoyloxy substituiert sind; und Salze davon.

9. Verbindungen der Formel I gemäss Anspruch 6, worin X Fluor, Chlor oder Methylsulfonyloxy bedeutet; $R_1$ und $R_2$ unabhängig voneinander Wasserstoff, Niederalkyl, $C_3$-$C_7$-Cycloalkyl oder Phenylniederalkyl bedeuten, oder $R_1$ und $R_2$ zusammen für einen Rest

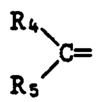

stehen, worin $R_4$ und $R_5$ unabhängig voneinander Wasserstoff, Niederalkyl, Halogenniederalkyl, $C_3$-$C_7$-Cycloalkyl, Phenylniederalkyl oder Phenyl bedeuten, oder worin $R_4$ Chinolinyl, 3-Hydroxy-5-(hydroxymethyl oder phosphonooxymethyl)-2-methyl-4-pyridyl bedeutet und $R_5$ für Wasserstoff steht, oder worin $R_4$ Carboxy oder Niederalkoxycarbonyl bedeutet und $R_5$ für Niederalkyl steht, oder worin $R_4$ und $R_5$ zusammen $-(CH_2)_4-$ oder $-(CH_2)_5-$ bedeuten; und $R_3$ für Wasserstoff, Niederalkyl, Niederalkenyl, Niederalkinyl oder Hydroxy steht; wobei Phenylgruppen unsubstituiert oder durch Niederalkyl, Trifluormethyl, Halogen, Hydroxy, Niederalkoxy und/oder Nitro substituiert sind; und Salze davon.

10. Verbindungen der Formel I gemäss Anspruch 6, worin X Fluor, Chlor oder Methylsulfonyloxy bedeutet, $R_1$ für Wasserstoff steht, $R_2$ Wasserstoff, $C_1$-$C_4$-Alkyl, Benzyl oder 2-Hydroxybenzyl bedeutet, oder $R_1$ und $R_2$ zusammen $C_5$-$C_6$-Cycloalkyliden, $C_1$-$C_4$-Alkyliden, Benzyliden, 2-Hydroxybenzyliden oder 3-Hydroxy-5-(hydroxymethyl oder phosphonooxymethyl)-2-methyl-4-pyridylmethyliden bedeuten, und $R_3$ für Wasserstoff, $C_1$-$C_4$-Alkyl, $C_2$-$C_4$-Alkinyl oder Hydroxy steht; und Salze davon.

11. 3-Benzylidenaminoxy-2-fluorpropylamin gemäss Anspruch 6 und pharmazeutisch annehmbare Salze davon.

12. 3-Cyclopentylidenaminoxy-2-hydroxypropylamin gemäss Anspruch 6 und pharmazeutisch annehmbare Salze davon.

13. 3-Aminoxy-2-fluorpropylamin gemäss Anspruch 6 und pharmazeutisch annehmbare Salze davon.

14. 3-Pyridoxalaminoxy-2-fluorpropylamin gemäss Anspruch 6 und pharmazeutisch annehmbare Salze davon.

15. 3-Amino-2-fluor-propyloxyimino-pyridoxalphosphat gemäss Anspruch 6 und pharmazeutisch annehmbare Salze davon.

16. 3-Aminoxy-2-methylsulfonyloxy-propylamin gemäss Anspruch 6 und pharmazeutisch annehmbare Salze davon.

17. 3-(2-Hydroxybenzylidenaminoxy)-2-fluorpropylamin gemäss Anspruch 6 und pharmazeutisch annehmbare Salze davon.

18. 3-(1-Methoxymethylethylidenaminoxy)-2-fluorpropylamin gemäss Anspruch 6 und pharmazeutisch annehmbare Salze davon.

19. 3-(1-Methyl-propylidenaminoxy)-2-hydroxypropylamin gemäss Anspruch 6 und pharmazeutisch annehmbare Salze davon.

20. 3-(2,2-Difluor-1-phenyl-ethylidenaminoxy)-2-fluor-propylamin gemäss Anspruch 6 und pharmazeutisch annehmbare Salze davon.

21. 4-(3-Amino-2-fluor-propyloxyiminomethyl)-chinolin gemäss Anspruch 6 und pharmazeutisch annehmbare Salze davon.

22. Pharmazeutisches Präparat enthaltend eine Verbindung gemäss Anspruch 6 und mindestens ein pharmazeutisch verwendbares Trägermaterial.

23. Eine Verbindung gemäss Anspruch 6 zur Anwendung in einem Verfahren zur therapeutischen Behandlung des tierischen oder menschlichen Körpers.

24. Eine Verbindung gemäss Anspruch 6 zur Verwendung als antitumorwirksames Mittel.

25. Verwendung von einer Verbindung gemäss Anspruch 6 zur Herstellung von pharmazeutischen Präparaten.

26. Verfahren zur Herstellung einer Verbindung der Formel I gemäss Anspruch 6, dadurch gekennzeichnet, dass man

(a) zur Herstellung von Verbindungen der Formel I, worin X für Halogen steht, eine Verbindung der Formel II

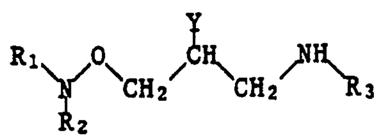

(II)

worin $R_1$, $R_2$ und $R_3$ die unter Formel I angegebene Bedeutung haben und Y eine in Halogen überführbare oder durch Halogen austauschbare Gruppe ist, mit einem Halogenierungsmittel umsetzt, oder

(b) zur Herstellung von Verbindungen der Formel I, worin X für Hydroxy steht, eine Verbindung der Formel III

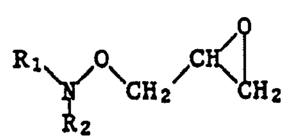

(III)

worin $R_1$ und $R_2$ die unter Formel I angegebene Bedeutung haben, mit Ammoniak bzw. einem Amin der Formel $NH_2R_3$, worin $R_3$ die unter Formel I angegebene Bedeutung hat, umsetzt, oder

(c) zur Herstellung von Verbindungen der Formel I, worin $R_1$ Wasserstoff bedeutet, in einer Verbindung der Formel IV

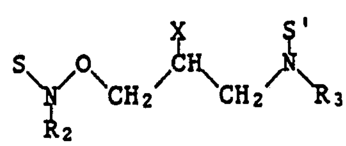

(IV)

worin $R_2$ und $R_3$ die unter Formel I angegebene Bedeutung haben und S und S′ unabhängig voneinander für eine Aminoschutzgruppe oder Wasserstoff stehen, wobei mindestens eine der Gruppen S und S′ eine Aminoschutzgruppe bedeutet, oder worin S und $R_2$ zusammen bzw. S′ und $R_3$ zusammen eine bivalente Aminoschutzgruppe bedeuten, die Aminoschutzgruppe(n) abspaltet, oder

(d) eine Verbindung der Formel V

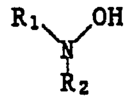

(V)

worin $R_1$ und $R_2$ die unter Formel I angegebene Bedeutung haben, mit einer Verbindung der Formel VI

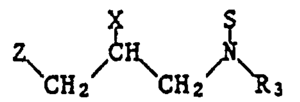

(VI)

worin $R_3$ und X die unter Formel I angegebene Bedeutung haben, Z Hydroxy oder eine nucleofuge Abgangsgruppe bedeutet und S eine Aminoschutzgruppe oder Wasserstoff bedeutet, umsetzt und gegebenenfalls die Aminoschutzgruppe abspaltet, oder
(e) zur Herstellung von Verbindungen der Formel I, worin $R_3$ Wasserstoff bedeutet, in einer Verbindung der Formel VII

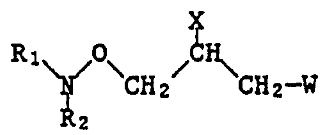

(VII)

worin $R_1$, $R_2$ und X die unter Formel I angegebene Bedeutung haben und W einen in Amino überführbaren Rest bedeutet, den Rest W in Amino überführt; oder
(f) zur Herstellung von Verbindungen der Formel I, worin $R_3$ Wasserstoff oder insbesondere Hydroxy oder Niederalkyl bedeutet, eine Verbindung der Formel VIII

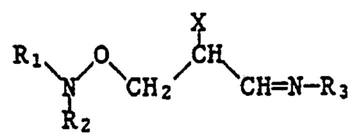

(VIII)

worin $R_1$, $R_2$ und X die unter Formel I angegebene Bedeutung haben und $R_3$ Wasserstoff, Hydroxy oder Niederalkyl bedeutet, reduziert;
und/oder wenn erwünscht, eine erhaltene Verbindung der Formel I in eine andere Verbindung der Formel S umwandelt, und/oder, wenn erwünscht, ein erhaltenes Salz in die freie Verbindung oder in ein anderes Salz um wandelt, und/oder, wenn erwünscht, eine erhaltene freie Verbindung in ein Salz umwandelt, und/oder ein erhaltenes Gemisch von isomeren Verbindungen der Formel I in die einzelnen Isomeren auftrennt.

27. Die nach den Verfahren gemäss Anspruch 26 erhältlichen Verbindungen.

Patentansprüche für folgende Vertragsstaaten: ES, GR

1. Verfahren zur Herstellung von Verbindungen der Formel I

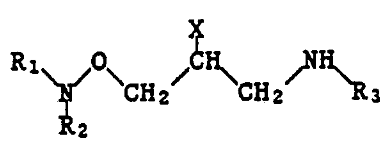

(I),

worin X Halogen, Hydroxy, Niederalkoxy, Acyloxy, Niederalkylsulfonyloxy oder Arylsulfonyloxy bedeutet; $R_1$ und $R_2$ unabhängig voneinander Wasserstoff, Niederalkyl, Cycloalkyl, Arylniederalkyl oder Hetarylniederalkyl bedeuten, oder $R_1$ und $R_2$ zusammen für einen Rest

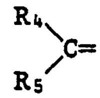

stehen, worin $R_4$ und $R_5$ unabhängig voneinander Wasserstoff, Niederalkyl, Hydroxyniederalkyl, Halogenniederalkyl, Niederalkoxyniederalkyl, Niederalkenyl, Niederalkinyl, Cycloalkyl, Arylniederalkyl, Hetarylniederalkyl, Aryl, Hetaryl, Carboxy oder Niederalkoxycarbonyl bedeuten, oder worin $R_4$ Polyhydroxyniederalkyl bedeutet und $R_5$ für Wasserstoff oder Hydroxymethyl steht, oder worin $R_4$ und $R_5$ zusammen $C_2$-$C_7$-Alkylen oder gegebenenfalls substituiertes Benzo-$C_4$-$C_6$-alkylen bedeuten; und $R_3$ für Wasserstoff, gegebenenfalls durch Hydroxy oder Halogen und/oder Aminoxy substituiertes Niederalkyl, Niederalkenyl, Niederalkinyl oder gegebenenfalls verethertes Hydroxy steht;
mit der Massgabe, dass, wenn X Hydroxy bedeutet und $R_1$ und $R_2$ zusammen für einen Rest

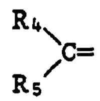

stehen, $R_3$ Wasserstoff bedeutet;
mit der Massgabe, dass, wenn X für Hydroxy steht und $R_3$ Wasserstoff bedeutet, die Reste $R_1$ und $R_2$ zusammen nicht 1,1-Dimethylmethyliden, 1-n-Butyliden oder Nitro-(furanyl oder imidazolyl)-methyliden bedeuten können;
mit der Massgabe, dass, wenn X für Hydroxy steht und $R_3$ Wasserstoff, Hydroxy oder Niederalkyl bedeutet, nicht beide Reste $R_1$ und $R_2$ Wasserstoff bedeuten können;
und mit der Massgabe, dass, wenn X Hydroxy bedeutet und $R_1$ und $R_2$ unabhängig voneinander Wasserstoff, Niederalkyl, Cycloalkyl, Arylniederalkyl oder Hetarylniederalkyl, nicht jedoch beide Wasserstoff, bedeuten, $R_3$ für Wasserstoff, Methyl, Ethyl oder n-Propyl steht; und Salzen davon, dadurch gekennzeichnet, dass man
(a) zur Herstellung von Verbindungen der Formel I, worin X für Halogen steht, eine Verbindung der Formel II

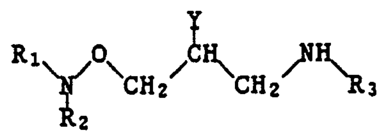

(II)

worin $R_1$, $R_2$ und $R_3$ die unter Formel I angegebene Bedeutung haben und Y eine in Halogen überführbare oder durch Halogen austauschbare Gruppe ist, mit einem Halogenierungsmittel umsetzt, oder
(b) zur Herstellung von Verbindungen der Formel I, worin X für Hydroxy steht, eine Verbindung der Formel III

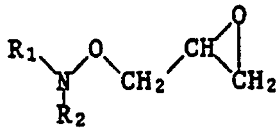

(III)

worin $R_1$ und $R_2$ die unter Formel I angegebene Bedeutung haben, mit Ammoniak bzw. einem Amin der Formel $NH_2R_3$, worin $R_3$ die unter Formel I angegebene Bedeutung hat, umsetzt, oder
(c) zur Herstellung von Verbindungen der Formel I, worin $R_1$ Wasserstoff bedeutet, in einer Verbindung der Formel IV

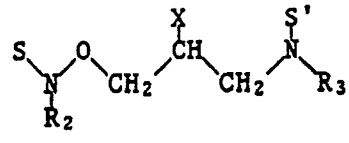

(IV)

worin $R_2$ und $R_3$ die unter Formel I angegebene Bedeutung haben und S und S′ unabhängig voneinander für eine Aminoschutzgruppe oder Wasserstoff stehen, wobei mindestens eine der Gruppen S und S′ eine Aminoschutzgruppe bedeutet, oder worin S und $R_2$ zusammen bzw. S′ und $R_3$ zusammen eine bivalente Aminoschutzgruppe bedeuten, die Aminoschutzgruppe(n) abspaltet, oder

(d) eine Verbindung der Formel V

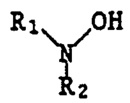
(V)

worin $R_1$ und $R_2$ die unter Formel I angegebene Bedeutung haben, mit einer Verbindung der Formel VI

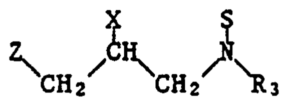
(VI)

worin $R_3$ und X die unter Formel I angegebene Bedeutung haben, Z Hydroxy oder eine nucleofuge Abgangsgruppe bedeutet und S eine Aminoschutzgruppe oder Wasserstoff bedeutet, umsetzt und gegebenenfalls die Aminoschutzgruppe abspaltet, oder

(e) zur Herstellung von Verbindungen der Formel I, worin $R_3$ Wasserstoff bedeutet, in einer Verbindung der Formel VII

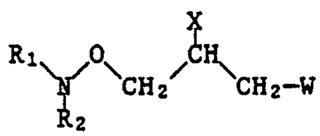
(VII)

worin $R_1$, $R_2$ und X die unter Formel I angegebene Bedeutung haben und W einen in Amino überführbaren Rest bedeutet, den Rest W in Amino überführt; oder

(f) zur Herstellung von Verbindungen der Formel I, worin $R_3$ Wasserstoff oder insbesondere Hydroxy oder Niederalkyl bedeutet, eine Verbindung der Formel VIII

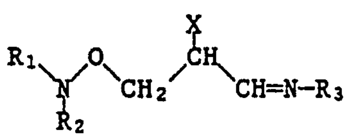
(VIII)

worin $R_1$, $R_2$ und X die unter Formel I angegebene Bedeutung haben und $R_3$ Wasserstoff, Hydroxy oder Niederalkyl bedeutet, reduziert;

und/oder wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz um wandelt, und/oder, wenn erwünscht, eine erhaltene freie Verbindung in ein Salz umwandelt, und/oder ein erhaltenes Gemisch von isomeren Verbindungen der Formel I in die einzelnen Isomeren auftrennt.

2. Verfahren gemäss Anspruch 1 zur Herstellung von Verbindungen der Formel I, worin X Halogen, Hydroxy, Niederalkoxy, Niederalkylsulfonyloxy oder Phenylsulfonyloxy bedeutet; $R_1$ und $R_2$ unabhängig voneinander Wasserstoff, Niederalkyl, $C_3$-$C_8$-Cycloalkyl oder Phenylniederalkyl bedeuten, oder $R_1$ und $R_2$ zusammen für einen Rest

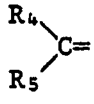

stehen, worin $R_4$ und $R_5$ unabhängig voneinander Wasserstoff, Niederalkyl, Halogenniederalkyl, $C_3$-$C_8$-Cycloalkyl, Phenylniederalkyl oder Phenyl bedeuten, oder worin $R_4$ 3-Hydroxy-5-(hydroxymethyl oder

phosphonooxymethyl)-2-methyl-4-pyridyl bedeutet und $R_5$ für Wasserstoff steht, oder worin $R_4$ 1,2,3,4-Tetrahydroxybutyl oder 1,2,3,4,5-Pentahydroxypentyl bedeutet und $R_5$ für Wasserstoff oder Hydroxymethyl steht, oder worin $R_4$ Chinolinyl, Carboxy oder Niederalkoxycarbonyl bedeutet und $R_5$ für Wasserstoff oder Niederalkyl steht, oder worin $R_4$ und $R_5$ zusammen $C_2$-$C_7$-Alkylen oder gegebenenfalls durch Cyano substituiertes Benzo-$C_4$-$C_6$-alkylen bedeuten; und $R_3$ für Wasserstoff, Niederalkyl, Niederalkenyl, Niederalkinyl, Niederalkoxy, Benzyloxy oder Hydroxy steht;
mit der Massgabe, dass, wenn X Hydroxy bedeutet und $R_1$ und $R_2$ zusammen für einen Rest

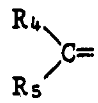

stehen, $R_3$ Wasserstoff bedeutet;
mit der Massgabe, dass, wenn X für Hydroxy steht und $R_3$ Wasserstoff bedeutet, die Reste $R_1$ und $R_2$ zusammen nicht 1,1-Dimethylmethyliden oder 1-n-Butyliden bedeuten können;
mit der Massgabe, dass, wenn X für Hydroxy steht und $R_3$ Wasserstoff, Hydroxy oder Niederalkyl bedeutet, nicht beide Reste $R_1$ und $R_2$ Wasserstoff bedeuten können;
und mit der Massgabe, dass, wenn X Hydroxy bedeutet und $R_1$ und $R_2$ unabhängig voneinander Wasserstoff, Niederalkyl, Cycloalkyl oder Phenylniederalkyl, nicht jedoch beide Wasserstoff, bedeuten, $R_3$ für Wasserstoff, Methyl, Ethyl oder n-Propyl steht;
wobei Phenylgruppen unsubstituiert oder durch Niederalkyl, Halogenniederalkyl, Halogen, Hydroxy, Niederalkoxy, Niederalkanoyloxy und/oder Nitro substituiert sind; und Salzen davon.

3. Verfahren gemäss Anspruch 1 zur Herstellung von Verbindungen der Formel I, worin X Fluor, Chlor, Hydroxy, Niederalkylsulfonyloxy oder Phenylsulfonyloxy bedeutet; $R_1$ und $R_2$ unabhängig voneinander Wasserstoff, Niederalkyl, $C_3$-$C_7$-Cycloalkyl oder Phenylniederalkyl bedeuten, mit der Massgabe, dass nicht beide Reste $R_1$ und $R_2$ Wasserstoff bedeuten können, wenn X für Hydroxy steht,
oder worin $R_1$ und $R_2$ zusammen für einen Rest

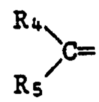

stehen, worin $R_4$ und $R_5$ unabhängig voneinander Wasserstoff, Niederalkyl, Halogenniederalkyl, $C_3$-$C_7$-Cycloalkyl, Phenylniederalkyl oder Phenyl bedeuten, oder worin $R_4$ Chinolinyl, 3-Hydroxy-5-(hydroxymethyl oder phosphonooxymethyl)-2-methyl-4-pyridyl bedeutet und $R_5$ für Wasserstoff steht, oder worin $R_4$ Carboxy oder Niederalkoxycarbonyl bedeutet und $R_5$ für Niederalkyl steht, oder worin $R_4$ und $R_5$ zusammen $-(CH_2)_4-$ oder $-(CH_2)_5-$ bedeuten; und $R_3$ für Wasserstoff steht;
mit der Massgabe, dass, wenn X für Hydroxy steht, die Reste $R_1$ und $R_2$ zusammen nicht 1,1-Dimethylmethyliden oder 1-n-Butyliden bedeuten können; wobei Phenylgruppen unsubstituiert oder durch Niederalkyl, Trifluormethyl, Halogen, Hydroxy, Niederalkoxy oder Niederalkanoyloxy substituiert sind; und Salzen davon.

4. Verfahren gemäss Anspruch 1 zur Herstellung von Verbindungen der Formel I, worin X Fluor, Chlor oder Methylsulfonyloxy bedeutet; $R_1$ und $R_2$ unabhängig voneinander Wasserstoff, Niederalkyl, $C_3$-$C_7$-Cycloalkyl oder Phenylniederalkyl bedeuten, oder $R_1$ und $R_2$ zusammen für einen Rest

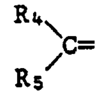

stehen, worin $R_4$ und $R_5$ unabhängig voneinander Wasserstoff, Niederalkyl, Halogenniederalkyl, $C_3$-$C_7$-Cycloalkyl, Phenylniederalkyl oder Phenyl bedeuten, oder worin $R_4$ Chinolinyl, 3-Hydroxy-5-(hydroxymethyl oder phosphonooxymethyl)-2-methyl-4-pyridyl bedeutet und $R_5$ für Wasserstoff steht, oder worin $R_4$ Carboxy oder Niederalkoxycarbonyl bedeutet und $R_5$ für Niederalkyl steht, oder worin $R_4$ und $R_5$ zusammen $-(CH_2)_4-$ oder $-(CH_2)_5-$ bedeuten; und $R_3$ für Wasserstoff, Niederalkyl, Niederalkenyl, Niederalkinyl oder Hydroxy steht; wobei Phenylgruppen unsubstituiert oder durch Niederalkyl, Trifluormethyl, Halogen, Hydroxy, Niederalkoxy und/oder Nitro substituiert sind; und Salzen davon.

5. Verfahren gemäss Anspruch 1 zur Herstellung von Verbindungen der Formel I, worin X Fluor, Chlor oder Methylsulfonyloxy bedeutet, $R_1$ für Wasserstoff steht, $R_2$ Wasserstoff, $C_1$-$C_4$-Alkyl, Benzyl oder 2-Hydroxybenzyl bedeutet, oder $R_1$ und $R_2$ zusammen $C_5$-$C_6$-Cycloalkyliden, $C_1$-$C_4$-Alkyliden, Benzyliden, 2-Hydroxybenzyliden oder 3-Hydroxy-5-(hydroxymethyl oder phosphonooxymethyl)-2-methyl-4-pyridylmethyliden bedeuten, und $R_3$ für Wasserstoff, $C_1$-$C_4$-Alkyl, $C_2$-$C_4$-Alkinyl oder Hydroxy steht; und Salzen davon.

6. Verfahren gemäss Anspruch 1 zur Herstellung von 3-Benzylidenaminoxy-2-fluorpropylamin und pharmazeutisch annehmbaren Salzen davon.

7. Verfahren gemäss Anspruch 1 zur Herstellung von 3-Cyclopentylidenaminoxy-2-hydroxypropylamin und pharmazeutisch annehmbaren Salzen davon.

8. Verfahren gemäss Anspruch 1 zur Herstellung von 3-Aminoxy-2-fluorpropylamin und pharmazeutisch annehmbaren Salzen davon.

9. Verfahren gemäss Anspruch 1 zur Herstellung von 3-Pyridoxalaminoxy-2-fluorpropylamin und pharmazeutisch annehmbaren Salzen davon.

10. Verfahren gemäss Anspruch 1 zur Herstellung von 3-Amino-2-fluorpropyloxyimino-pyridoxalphosphat und pharmazeutisch annehmbaren Salzen davon.

11. Verfahren gemäss Anspruch 1 zur Herstellung von 3-Aminoxy-2-methylsulfonyloxy-propylamin und pharmazeutisch annehmbaren Salzen davon.

12. Verfahren gemäss Anspruch 1 zur Herstellung von 3-(2-Hydroxybenzylidenaminoxy)-2-fluorpropylamin und pharmazeutisch annehmbaren Salzen davon.

13. Verfahren gemäss Anspruch 1 zur Herstellung von 3-(1-Methoxymethylethylidenaminoxy)-2-fluorpropylamin und pharmazeutisch annehmbaren Salzen davon.

14. Verfahren gemäss Anspruch 1 zur Herstellung von 3-(1-Methyl-propylidenaminoxy)-2-hydroxypropylamin und pharmazeutisch annehmbaren Salzen davon.

15. Verfahren gemäss Anspruch 1 zur Herstellung von 3-(2,2-Difluor-1-phenyl-ethylidenaminoxy)-2-fluorpropylamin und pharmazeutisch annehmbaren Salzen davon.

16. Verfahren gemäss Anspruch 1 zur Herstellung von 4-(3-Amino-2-fluorpropyloxyiminomethyl)-chinolin und pharmazeutisch annehmbaren Salzen davon.

17. Verfahren zur Herstellung eines pharmazeutischen Präparats, dadurch gekennzeichnet, dass man eine nach dem Verfahren gemäss Anspruch 1 erhältliche Verbindung mit einem pharmazeutischen Trägermaterial verarbeitet.

Europäisches Patentamt

# EUROPÄISCHER RECHERCHENBERICHT

Nummer der Anmeldung

EP 89 81 0859

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| P,A | CHEMICAL ABSTRACTS, Band 110, Nr. 3, 16. Januar 1989, Seite 234, Zusammenfassung Nr. 20327e, Columbus, Ohio, US; A.R. KHOMUTOV et al.: "Polyfunctional O-substituted hydroxylamines for specific inhibition of enzymes and modification of nucleic acids", & F.E.C.S. INT. CONF. CHEM. BIOTECHNOL. BIOL. ACT. NAT. PROD. [PROC.], 3RD 1985 (Pub. 1987) 4, 214-18 | 1 | A 61 K 31/15<br>C 07 C 251/58<br>C 07 C 239/20<br>C 07 C 309/66<br>C 07 D 213/69<br>C 07 D 215/12<br>C 07 F 9/58<br>C 07 H 5/04 |
| A | CHEMICAL ABSTRACTS, Band 100, Nr. 17, 23. April 1984, Seite 611, Zusammenfassung Nr. 138693t, Columbus, Ohio, US; N. AMLAIKY et al.: "Synthesis of beta-blocking oximes. 3. Effect of terminal amine on beta-selectivity", & EUR. J. MED. CHEM. - CHIM. THER. 1983, 18(5), 437-9 | 6 | |
| | | | **RECHERCHIERTE SACHGEBIETE (Int. Cl.5)** |
| A | EP-A-0 288 055 (MERRELL DOW) | 1 | A 61 K 31/00<br>C 07 C 251/00<br>C 07 C 239/00<br>C 07 C 309/00<br>C 07 D 213/00<br>C 07 D 215/00<br>C 07 F 9/00<br>C 07 H 5/00 |
| A | EP-A-0 229 658 (MERRELL DOW) | 1 | |
| A | EP-A-0 184 112 (CHUGAI SEIYAKU K.K.) | 6 | |
| A | WO-A-8 402 908 (N. AMLAIKY et al.) | 6 | |
| X | DE-A-2 651 083 (HOECHST AG)<br>* Verbindungen 24,25 * | 6,7,22, 23,26, 27 | |

---  -/-

Der vorliegende Recherchenbericht wurde für alle Patentansprüche erstellt

| Recherchenort | Abschlußdatum der Recherche | Prüfer |
|---|---|---|
| DEN HAAG | 18-12-1989 | WELLS A.G. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung

EP 89 81 0859

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | JOURNAL OF MEDICINAL CHEMISTRY, Band 28, Nr. 2, Februar 1985, Seiten 153-160, American Chemical Society; B. MACCHIA et al.: "An interdisciplinary approach to the design of new structures active at the beta-adrenergic receptor. Aliphatic oxime erther derivatives" * Seite 155, Verbindung 16 * ----- | 6,7,26, 27 | |

RECHERCHIERTE SACHGEBIETE (Int. Cl.5)

Der vorliegende Recherchenbericht wurde für alle Patentansprüche erstellt

| Recherchenort | Abschlußdatum der Recherche | Prüfer |
|---|---|---|
| DEN HAAG | 18-12-1989 | WELLS A.G. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

# ANHANG ZUM EUROPÄISCHEN RECHERCHENBERICHT ÜBER DIE EUROPÄISCHE PATENTANMELDUNG NR.

EP 89 81 0859

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am 23/01/90
Diese Angaben dienen nur zur Unterrichtung und erfolgen ohne Gewähr.

| Im Recherchenbericht angeführtes Patentdokument | Datum der Veröffentlichung | Mitglied(er) der Patentfamilie | Datum der Veröffentlichung |
|---|---|---|---|
| EP-A- 0288055 | 26-10-88 | AU-A- 1502388 | 27-10-88 |
| | | JP-A- 63280019 | 17-11-88 |
| | | ZA-A- 8802719 | 17-10-88 |
| EP-A- 0229658 | 22-07-87 | US-A- 4707498 | 17-11-87 |
| | | AU-B- 589245 | 05-10-89 |
| | | AU-A- 6747787 | 16-07-87 |
| | | JP-A- 62167744 | 24-07-87 |
| EP-A- 0184112 | 11-06-86 | JP-A- 61267548 | 27-11-86 |
| | | JP-A- 61267550 | 27-11-86 |
| WO-A- 8402908 | 02-08-84 | FR-A,B 2539413 | 20-07-84 |
| | | AU-A- 2415884 | 15-08-84 |
| | | EP-A- 0131595 | 23-01-85 |
| DE-A- 2651083 | 18-05-78 | AT-B- 363452 | 10-08-81 |
| | | AU-B- 520996 | 11-03-82 |
| | | AU-A- 3043377 | 17-05-79 |
| | | BE-A- 860614 | 08-05-78 |
| | | CA-A- 1081242 | 08-07-80 |
| | | FR-A,B 2370028 | 02-06-78 |
| | | GB-A- 1540028 | 07-02-79 |
| | | JP-A- 53068711 | 19-06-78 |
| | | NL-A- 7712291 | 11-05-78 |
| | | SE-B- 441265 | 23-09-85 |
| | | SE-A- 7712630 | 09-05-78 |
| | | US-A- 4404384 | 13-09-83 |

EPO FORM P0459